# EUROPEAN PATENT SPECIFICATION

(11) **EP 1 730 179 B1**
(45) Date of publication and mention of the grant of the patent: **28.04.2010**
(21) Application number: 05722070.9
(22) Date of filing: 22.03.2005
(51) Int. Cl.: C07K 14/335

(54) **NOVEL MANNOSE-SPECIFIC ADHESINS AND THEIR USE**
MANNOSE-SPEZIFISCHE ADHESINEN UND DEREN VERVENDUNG
NOUVELLES ADHESINES SPECIFIQUES DU MANNOSE ET UTILISATIONS DE CELLES-CI

(30) Priority: 23.03.2004 EP 04075945
(43) Date of publication of application: 13.12.2006
(73) Proprietor: Friesland Brands B.V., 7943 PE Meppel (NL)
(72) Inventor: PRETZER, Gabriele, NL-6826 PW Arnhem (NL); SNEL, Johannes, NL-6703 GX Wageningen (NL); BRON, Peter, Allard, NL-6701 DB Wageningen (NL); DE VOS, Willem, Meindert, NL-6721 SJ Bennekom (NL); KLEEREBEZEM, Michiel, NL-6716 GG Ede (NL)
(74) Representative: van Westenbrugge, Andries
(86) International application number: PCT/NL2005/000216
(87) International publication number: WO 2005/090396

(56) References cited:
- US-A- 6 159 465
- AHRNE S ET AL: "The normal Lactobacillus flora of healthy human rectal and oral mucosa" JOURNAL OF APPLIED MICROBIOLOGY, vol. 85, no. 1, July 1998 (1998-07), pages 88-94, XP002330159 ISSN: 1364-5072
- ADLERBERTH I ET AL: "A Mannose-Specific Adherence Mechanism in Lactobacillus plantarum Conferring Binding to the Human Colonic Cell Line HT-29" APPLIED AND ENVIRONMENTAL MICROBIOLOGY, WASHINGTON,DC, US, vol. 62, no. 7, July 1996 (1996-07), pages 2244-2251, XP002979206 ISSN: 0099-2240
- KLEEREBEZEM MICHIEL ET AL: "Complete genome sequence of Lactobacillus plantarum WCFS1" PROCEEDINGS OF THE NATIONAL ACADEMY OF SCIENCES OF USA, NATIONAL ACADEMY OF SCIENCE. WASHINGTON, US, vol. 100, no. 4, 18 February 2003 (2003-02-18), pages 1990-1995, XP002285325 ISSN: 0027-8424
- DATABASE EMBL Cell Surface Protein 1 June 2003 (2003-06-01), XP002297276 retrieved from EBI Database accession no. Q88XH5

## Description

### Field of the invention

The present invention relates to novel mannose-specific adhesins, and variants thereof, as well as nucleic acid sequences encoding these. The invention also relates to host cells comprising one or more mannose-specific adhesins (or variants thereof), as well as foods, food ingredients, and pharmaceutical or nutraceutical compositions comprising one or more of the mannose-specific adhesins (or variants thereof) and/or comprising host cells capable of expressing these, and their use in treating, preventing or delaying bacterial infection. Further, screening methods suitable for identifying bacteria strains with probiotic properties are provided.

### Background of the invention

Harmless commensal or even beneficial microorganisms (referred to as probiotic microorganisms) such as lactobacilli and other lactic acid bacteria commonly populate human and animal body cavities. For example, many strains of *Lactobacillus,* and related genera of lactic acid bacteria with a longstanding tradition in food industry, are present in large numbers on gastrointestinal mucosa together with other intestinal organisms (Mitsuoka, 1992 in The Lactic Acid Bacteria in Health and Disease, Volume I., E.B.J.B.Wood, Elsevier Science Publishing Ltd. pp 69-114; Ahrne et al. J Appl Microbiol. 1998 Jul;85(1): 88-94; Vaughan EE, et al. Antonie Van Leeuwenhoek. 2002 Aug;82(1-4):341-52; Reid et al. Clin Microbiol Rev. 2003 Oct; 16(4):658-72; Reid et al. J Clin Gastroenterol. 2003 Aug;37(2):105-18; Mercenier et al. Curr Pharm Des. 2003;9(2):175-91). These beneficial microorganisms are believed to play an important role in human and animal health by interfering with the invasion and colonisation of potential pathogens of the gastrointestinal tract. One of the mechanisms by which interference is believed to be achieved is competitive exclusion of pathogens by the beneficial microorganisms through occupation of common receptors on the mucosal surfaces. However, little is still known about the exact mechanisms through which beneficial bacterial species become established and persist in the indigenous microflora and how they inhibit or reduce attachment and colonisation of pathogenic bacteria.

Bacterial colonisation of mucosal tissue is generally mediated by adhesins, which are proteins present on the surface of both pathogenic and beneficial bacteria. Adhesins are thought to be responsible for the recognition and binding to specific receptors on the host cells, thereby playing a role in bacterial attachment and colonisation. In many instances, adhesins are localised on hair-like appendages of bacteria (pili or fimbriae), although they may also be directly associated with the bacterial cell surface (non-pilus adhesins) (see for example Soto and Hultgren, J. of Bacteriology 1999, Vol. 181(4), p1059-1071).

Various pathogenic Gram-negative bacteria, including enteropathogenic *Escherichia coli* (EPEC), display mannose-specific adhesion properties that play a role during the infection process. For example, the type 1 fimbriae of uropathogenic *E. coli* strains comprise the adhesin FimH, which binds to mannose ligands present on the host tissue surfaces and appears to be involved in the establishment of kidney and bladder infections (Krogfelt et al. 1990 Infect. Immun. 58:1995-1998).

Mannose-specific adhesion is a phenotype which is also found in several lactic acid bacteria. Notably, it has been shown that certain strains of *Lactobacillus plantarum* (in particular strain 299v) are capable of adhering specifically to mannoside containing sugar moieties on the surface of human epithelial cells (Adlerberth et al., 1996, Applied and Environmental Microbiology, p2244-2251 and WO96/29083). It has been speculated that a proteinaceous bacterial structure is involved in this phenotype, as treatment with proteinase K could abolish the phenotype (Adlerberth et al., supra). Moreover, several lines of evidence show that this phenotypic feature of *L. plantarum* could be involved in the inhibition of EPEC infection by competitive exclusion (Michail & Abernathy, Pediatr. Gastroenterol. Nutr. 2002 35:350-5 and Mangell et al. Dig Dis Sci. 2002, 47, p511-6). This functional, phenotypic characteristic represents one of the interactions of *L. plantarum* and the human host that is functionally meaningful and experimentally testable. Various functional tests can be used to establish whether an *L. plantarum* strain possesses mannose-specific adhesion properties. Examples of such well-known functional tests are the mannose-sensitive agglutination test of yeast or erythrocytes, the direct binding of bacteria to D-mannose immobilised on agarose beads or other surfaces, cell binding (e.g. epithelial cells or cell lines) and the like.

Despite the observed phenotypic effect (mannose-specific adhesion), no molecular data are available to explain the mannose-specific adhesion properties of *L. plantarum* and therefore, it remains unknown whether indeed one or more proteins are involved in this interaction, and if so, which protein or proteins (and which gene or genes) of *L. plantarum* is (are) involved in this phenotypic trait. Moreover, although the complete 3.3 Mbp genome sequence of *L. plantarum* strain WCFS1 has been determined (Kleerebezem et al., Proc. Natl. Acad. Sci. USA, 2003, 18:1990-5), *in silico* analysis does not allow for the identification of the gene involved in this function. The inability to correlate gene sequences data with *in vivo* function is a common problem faced nowadays, as sequence homology or similarity to sequences with known function (if these are available at all) can at best provide a hypothetical function. Moreover, it is common knowledge that a significant fraction of sequenced genomes (usually about one third) cannot even be functionally annotated since these sequences are either unique, or only have homologues of unknown function. This is clearly exemplified by the finding that for the *L. plantarum* WCFS1 genome, 588 genes (~19 %) were annotated as conserved hypothetical proteins of unknown function, while 344 genes (~11 %) were annotated that do not have a homologue in public sequence databases (Kleerebezem et al. 2003, *supra).*

An understanding of which protein(s) are involved in determining mannose-specific adhesion in *L. plantarum* is essential for a) transferring this beneficial property to other prokaryotic or eukaryotic cells, b) improving or modulating the adhesion efficiency and/or specificity of microorganisms, c) developing pharmaceutical or nutraceutical compositions which reduce or prevent pathogen infection (e.g. vaccines) and d) for setting up easier methods for screening microorganisms, in particular bacterial collections, for the presence/absence of mannose-specific adhesion.

Microorganisms, especially probiotic bacteria, which have an improved ability to colonise human body cavities and thereby are more efficient in excluding or reducing colonisation by pathogenic microorganism, clearly have great medical value in the treatment and prophylaxis of diseases caused by pathogen infections. Similarly, the purified protein responsible for mannose-specific adhesion may in itself be of great value for the development of novel vaccines (see Wizemann et al. 1999, Emerging Infectious Diseases Vol. 5(3), p395-403) and therapeutic or prophylactic pharmaceuticals / nutraceuticals.

The elucidation of the gene(s) and protein(s) responsible for mannose-specific adhesion is also useful for improving the attachment and persistence of microorganisms which are used as delivery vehicles for e.g. vaccines, such as lactic acid bacteria as reviewed by Wells et al. (1996 Antonie Van Leeuwenhoek 70: 317-330) and Pouwels et al. (1998 Int. J. Food Microbiol. 41: 155-167).

### Description of the invention

### Definitions

"Mannose-specific adhesion" or "mannose-sensitive adhesion" refers to the ability of a cell, in particular a microorganism, especially a bacterium, to adhere to mannose comprising ligands, such as D-mannose or methyl-α-D-mannoside. Mannose-specific adhesion can be determined by various tests, such as the yeast agglutination test, the hemagglutination test, the binding of cells to immobilised mannose (e.g. mannose coated beads), the mannose-sensitive binding to human cell lines, and others as available in the art and as described herein. When referring to a protein or polypeptide "mannose-specific adhesion" refers to the ability of the protein to confer, or to significantly enhance, this phenotype when the nucleic acid encoding the protein is expressed in a host cell, especially a bacterial cell.

"Lactic acid bacteria" and "lactic acid producing bacteria", is used herein interchangeably and refers to bacteria, which produce lactic acid as an end product of fermentation, such as, but not limited to, bacteria of the genus *Lactobacillus, Streptococcus, Lactococcus, Oenococcus, Leuconostoc, Pediococcus, Carnobacterium, Propionibacterium, Enterococcus* and *Bifidobacterium.*

"Probiotics" or "probiotic strain(s)" refers to strains of live microorganisms, preferably bacteria, which have a beneficial effect on the host when ingested (e.g. enterally or by inhalation) by a subject. A "subject" refers herein to a human or non-human animal, in particular a vertebrate.

The term "LPXTG-like anchoring motif' refers to a cell wall anchoring region commonly found in surface-anchored proteins of Gram-positive bacteria. These motifs can be used to attach proteins or hybrid proteins (e.g. covalently) to the cell surface of Gram-positive bacteria, as described by Leenhouts et al. (Antonie van Leeuwenhoek 1999, 76(1-4):367-76). The *L. plantarum srtA* gene encodes a sortase, which is involved in maturation and anchoring of proteins comprising an LPXTG-like anchoring motif to the cell wall (Navarre and Schneewind, 1999, Microbial Mol. Biol. Rev. 63:174-229).

The term "gene" means a DNA fragment comprising a region (transcribed region), which is transcribed into an RNA molecule (e.g. an mRNA) in a cell, operably linked to suitable regulatory regions (e.g. a promoter). A gene may thus comprise several operably linked fragments, such as a promoter, a 5' leader sequence comprising e.g. sequences involved in translation intitiation, a (protein) coding region and a 3'non-translated sequence comprising e.g. transcription termination sites. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide. In one embodiment the coding sequence is preferably preceded by a nucleic acid sequence encoding a secretion signal, so that the encoded protein or peptide is secreted out of the cell. The coding sequence is preferably in sense-orientation and encodes a desired, biologically active protein or peptide.

A "chimeric" (or recombinant) gene refers to any gene, which is not normally found in nature in a species, in particular a gene in which one or more parts of the nucleic acid sequence are present that are not associated with each other in nature. For example the promoter is not associated in nature with part or all of the transcribed region or with another regulatory region. The term "chimeric gene" is understood to include expression constructs in which a promoter or transcription regulatory sequence is operably linked to one or more coding sequences.

A "transcription regulatory sequence" is herein defined as a nucleic acid sequence that is capable of regulating the rate of transcription of a (coding) sequence operably linked to the transcription regulatory sequence. A transcription regulatory sequence as herein defined will thus comprise all of the sequence elements necessary for initiation of transcription (promoter elements), for maintaining and for regulating transcription, including e.g. attenuators or enhancers. Although mostly the upstream (5') transcription regulatory sequences of a coding sequence are referred to, regulatory sequences found downstream (3') of a coding sequence are also encompassed by this definition.

As used herein, the term "operably linked" refers to a linkage of polynucleotide elements in a functional relationship. A nucleic acid is "operably linked" when it is placed into a functional relationship with another nucleic acid sequence. For instance, a promoter, or rather a transcription regulatory sequence, is operably linked to a coding sequence if it affects the transcription of the coding sequence. Operably linked means that the DNA sequences being linked are typically contiguous and, where necessary to join two protein encoding regions, contiguous and in reading frame.

The terms "signal sequence", "signal peptide" and "secretory leader" are used interchangeably and refer to a short (usually about 15-60 amino acids), continuous stretch of amino acids at the amino-terminus of secreted and membrane-bound polypeptides, which directs their delivery to various locations outside the cytosol. Signal sequences usually contain a hydrophobic core of about 4-15 amino acids, which is often immediately preceded by a basic amino acid. At the carboxyl-terminal end of the signal peptide there are a pair of small, uncharged amino acids separated by a single intervening amino acid that defines the signal peptide cleavage site (von Heijne, 1990). Despite their overall structural and functional similarities, native signal peptides do not have a consensus sequence.

The term "body cavity of a subject" is understood to mean a cavity in the body of a subject, in particular a vertebrate, that is externally accessible and that is usually lined with a mucosal surface. Examples of such cavities include the mouth, the nasopharynx, the upper and lower gastrointestinal tract, the rectum, the vagina and the non-mammalian counterparts of these mammalian cavities.

A "nucleic acid construct" is herein understood to mean a man-made nucleic acid molecule resulting from the use of recombinant DNA technology. The term "nucleic acid construct" therefore does not include naturally occurring nucleic acid molecules although a nucleic acid construct may comprise (parts of) naturally occurring nucleic acid molecules. A chimeric gene as defined above is an example of a nucleic acid construct, in particular it is an example of an expression construct. The term "vector" as used herein refers to a nucleic acid construct used for introducing exogenous DNA into host cells, such as for example bacterial cells. A plasmid vector thus at least comprises a replicon and a DNA segment that may be used for insertion of the exogenous DNA into the vector by recombinant techniques, preferably without interfering with the plasmids capability to replicate. Vectors usually comprise further genetic elements to facilitate their use in molecular cloning, such as e.g. selectable markers, multiple cloning sites and the like (see below). An "expression vector" as used herein refers to any cloning vector, in which a promoter and other regulatory elements are present to enable transcription of inserted exogenous DNA (e.g. one or more chimeric genes) when the expression vector is present in a suitable environment. The expression vector may be a plasmid-based vector. A "shuttle vector" refers to a plasmid vector that is capable of replication and stable maintenance in at least two different host organism, e.g. two organisms of different species or different genera. For this capability the shuttle vector may rely on a single broad host-range replicon but usually a shuttle vector will comprise different replicons for different host-organisms or different groups of host-organisms.

A "host cell" or a "recombinant host cell" are terms referring to a new individual cell (or organism) arising as a result of at least one nucleic acid molecule, especially comprising a gene encoding a desired protein, having been introduced into said cell. The host cell differs from naturally occurring cell in that they comprise one or more nucleic acid molecules, which have been introduced by either recombinant DNA techniques or by "natural" DNA transfer techniques, such as bacterial conjugation, followed by selection of recombinants at a molecular, a phenotypic and/or a functional level. The recombinant host cell may thus comprising a (man made) nucleic acid construct within its cell(s), in particular one or more chimeric genes. The host cell preferably contains the nucleic acid construct as an extrachromosomally (episomal) replicating molecule, or alternatively and more preferably, integrated into its genome. The latter has the advantage of greater genetic stability of the introduced DNA. It is immaterial by what method the nucleic acid construct is introduced into the host cell(s). Suitable transformation methods for introducing nucleic acid constructs into cells (prokaryotic or eukaryotic cells), such as e.g. electroporation, are available to a skilled person. Alternatively, the recombinant host cell may comprise one or more additional nucleic acid molecules, which were introduced by "natural" DNA transfer methods, such as bacterial conjugation, followed by targeted selection of cells in which the nucleic acid molecule(s) are introduced (and either integrated into the genome by e.g. homologous or non-homologous recombination or present on extrachromosomally replicating molecules, such as plasmids). Such a host cell is distinguished from a natural occurring cell by either the presence of additional copies of a specific gene (e.g. a change from 1 copy to more than one copy) compared to the non-recombinant cell or by the presence of one or more genes which were not previously present in the non-recombinant cell. In both cases, the introduced copy or copies of the gene(s) leads to a significant change of the phenotype of the host cell. If no copy was present in the non-recombinant cell, the conjugative transfer leads to the desired gain-of-function phenotype (e.g. mannose-specific adhesion) due to the expression of the transferred gene. If one or more copies were already present, the additional copy (or copies) leads to a enhancement of the desired function (e.g. enhanced mannose-specific adhesion) due to the enhanced production of the protein encoded by said gene. The recombinant host cell may be a eukaryotic or prokaryotic cell, in particular a microorganism (referred to as a "recombinant microorganism"), especially a bacterium (referred to as a "recombinant bacterium").

"Food-grade" micro-organisms are in particular organisms, which are considered as not harmful, when ingested by a human or animal subject, and which are acceptable as food ingredient.

The term "selectable marker" is a term familiar to one of ordinary skill in the art and is used herein to describe any genetic entity which, when expressed, can be used to select for a cell or cells containing the selectable marker. Selectable marker gene products confer for example antibiotic resistance. Genes conferring resistance to antibiotics such as kanamycin, rifampicin, erythromycin, actinomycin, chloramphenicol, tetracyclines, or other antimicrobial compounds like nisin and lactacin F are generally known in the art. The term "reporter" may be used interchangeably with marker, although it is mainly used to refer to visible markers, such as green fluorescent protein (GFP). Selectable markers may be dominant or recessive and they may further be bidirectional.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically (but not necessarily) be operably linked to another (heterologous) promoter sequence or, if applicable, another (heterologous) secretory signal sequence and/or terminator sequence than in its natural environment. Such regulatory sequences may also be homologous to the host cell. In this context, the use of only "homologous" sequence elements allows the construction of "self-cloned" genetically modified organisms (GMO's) (self-cloning is defined herein as in European Directive 98/81/EC Annex II; see also below herein). When used to indicate the relatedness of two nucleic acid sequences the term "homologous" means that one single-stranded nucleic acid sequence may hybridise to a complementary single-stranded nucleic acid sequence. The degree of hybridisation may depend on a number of factors including the amount of identity between the sequences and the hybridisation conditions such as temperature and salt concentration as discussed later.

"Stringent hybridisation conditions" can also be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100nt) are for example those which include at least one wash in 0.2X SSC at 63°C for 20min, or equivalent conditions. Stringent conditions for DNA-DNA hybridisation (Southern blots using a probe of e.g. 100nt) are for example those which include at least one wash (usually 2) in 0.2X SSC at a temperature of at least 50°C, usually about 55°C, for 20 min, or equivalent conditions.

The term "substantially identical", "substantial identity" or "essentially similar" or "essential similarity" means that two peptide or two nucleotide sequences, when optimally aligned, such as by the programs GAP or BESTFIT using default parameters, share at least a certain minimal percentage of sequence identity, as defined herein below. GAP uses the Needleman and Wunsch global alignment algorithm to align two sequences over their entire length, maximizing the number of matches and minimises the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992).

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". Sequences according to the invention

The present inventors were able to identify and isolate the nucleic acid sequence (SEQ ID NO: 2) from *L. plantarum,* which encodes a protein (SEQ ID NO: 1) responsible for mannose-specific adhesion of *L. plantarum.* This protein is referred to as "mannose-specific adhesin". This is the first mannose-specific adhesin isolated and characterised from a gram-positive bacterium. Knock-out mutants of *L. plantarum* WCFS1 (lacking a functional copy of the mannose-specific adhesion gene) completely lost the ability to agglutinate yeast in the yeast agglutination test. In contrast, overexpression of the mannose-specific adhesion gene in the wild type WCFS1 strain resulted in a significant enhancement of the mannose-specific agglutination of the recombinant strain. The experiments prove that the mannose-specific adhesin is responsible for conferring mannose-specific agglutination and adherence to cells comprising mannose molecules on the cell surface.

The mannose-specific adhesin is 1010 amino acids in length and comprises several repeated domains at the N- and C-terminal parts (residues 120-220, and residues 700-915, respectively). According to signaIP-HMM prediction, the amino acid sequence has a predicted N-terminal signal peptide for secretion by the Sec-dependent pathway, from residue 1 to 45 (http://www.cbs.dtu.dk/services/SignalP). Moreover, at the C-terminal the motif LPQTN is present, which is an LPXTG-like anchoring motif typically found in gram-positive cell-wall anchored surface proteins. This motif comprises a proteolytic cleavage site recognised and cleaved by the sortase enzyme (or surface protein transpeptidase), in *L. plantarum* encoded by the *srtA* gene (SEQ ID NO: 3 and SEQ ID NO: 4). However, enzymes performing similar function are also found in other gram-positive bacteria (see Navarre and Schneewind, 1999, supra).

Homology searches reveals domain-specific similiarity of mannose-specific adhesin (lp_1229) to several surface proteins of different lactobacilli and staphylococci, and includes a protein of Lactobacillus reuteri that has been shown to be involved in mucus binding properties displayed by this species (Roos and Jonsson, Microbiology. 2002, 148:433-42). The sequence similarities to L.reuteri are limited to the C-terminal repeat region of the lp_1229 mannose-specific adhesin. Residues 265-515 have strong similarity to a domain of large multi-domain surface proteins of Staphylococcus aureus (NCBI codes Q8NUJ3, Q8VQ99, Q99QY4) and S. epidermidis (NCBI code Q8CMU7; hemagglutinin protein), which also have a signal peptide, LPxTG-anchor and very large repeat regions. This domain is a member of the concanavalin A-like lectin/glucanase family (Interpro code IPR008985; structure superfamily code SSF49899), for which several 3-dimensional structures are known. Members of this family are found in a wide range of species, but exhibit the common property of reversibly binding to specific complex carbohydrates. Some bacterial and fungal beta-glucanases have this Con A-like domain, and carry out the acid hydrolysis of beta-glucans found in microorganisms and plants. There are many Con A-like domains found in proteins involved in cell recognition and adhesion. Hence, the presence of a carbohydrate-binding domain and additional similarity to a mucus-binding protein corroborate the role of this lp_1229 mannose-specific adhesin in interactions with the host or host-derived molecules.

In one embodiment, the invention provides isolated mannose-specific adhesin proteins and nucleic acid sequences encoding these. Provided is the mannose-specific adhesin of SEQ ID NO: 1 and the nucleic acid sequence encoding it, SEQ ID NO: 2. The protein or nucleic acid (DNA or RNA) may be either isolated from natural sources (such as various *L. plantarum* strains), or may be synthetically made *de novo* using chemical amino acid or nucleic acid synthesis. The mannose-specific adhesin, or functional variants thereof, may also be obtained from recombinant host cells overexpressing the mannose-specific adhesin (or a functional variant thereof), as described below.

Also provided are active variants of the mannose-specific adhesin protein of SEQ ID NO: 1, as well as nucleic acid sequences encoding these variants. Variants can be easily generated using methods known in the art, such as but not limited to amino acid substitutions or deletions (generating for example functional fragments), *de novo* chemical synthesis of peptides or mutagenesis- or gene-shuffling techniques, and the like. Variants may be tested for retention of functionality (mannose adhesion in the various assays described herein). Variants can also be identified and isolated from natural sources, such as other bacterial strains, using for example nucleic acid hybridisation techniques (e.g. Southern blot, Northern blot), and especially encompasses DNA sequences which hybridise to SEQ ID NO: 2 under stringent hybridisation conditions (defined above). It is clear that there are many approaches in the art to isolate variants of SEQ ID NO: 1 or SEQ ID NO: 2. An alternative to the use of nucleic acid hybridisation is for example the use of specific or degenerate PCR primers, designed to hybridise to specific regions of SEQ ID NO: 2 (or its complement strand) and to amplify variants using PCR technology. Substantially identical sequences can also be identified *in silico,* by using publicly available algorithms and databases such as BLAST, FASTA and the like, using SEQ ID No's 1 or 2 (or fragments or variants thereof) as input sequence. Sequences identified in this way can then either be cloned using standard molecular biology techniques or synthesised chemically using for example an oligonucleotide synthesiser as supplied e.g. by Applied Biosystems Inc. (Fosters, CA, USA).

Variants of the mannose-specific adhesin as defined herein include peptides with amino acid sequences with at least, 60%, 75%, 80%, 90%, 95% or 99% sequence identity to SEQ ID NO: 1 (as determined using the GAP program and default parameters), which retain the ability to confer or improve mannose-specific adhesion, as well as the nucleic acid sequences encoding these.

It is clear that due to the degeneracy of the genetic code various nucleic acid sequences may encode the same amino acid sequence. Thus, codons may be replaced, without changing the amino acid sequence of the protein. Similarly, some amino acids can be substituted by other equivalent amino acids without significantly changing the activity of the protein (at least not in a negative way). For example conservative amino acid substitutions within the categories basic (e.g. Arg, His, Lys), acidic (e.g. Asp, Glu), nonpolar (e.g. Ala, Val, Trp, Leu, Ile, Pro, Met, Phe) or polar (e.g. Gly, Ser, Thr, Tyr, Cys, Asn, Gln) fall within the scope of the invention as long as the mannose-specific adhesion property of the protein is not decreased by such changes. In addition non-conservative amino acid substitutions fall within the scope of the invention as long as the mannose-specific adhesion property of the protein is not decreased by such changes.

Variants of SEQ ID NO: 2 comprise nucleic acid sequences which have at least , 60%, 75%, 80%, 90%, 95%, or 99% sequence identity to SEQ ID NO: 2 (as determined using the GAP program and default parameters) and encode a protein which retains the ability to bind mannose specifically, i.e. to confer a mannose-specific adhesion phenotype to the host cell in which it is expressed or, if the host cell already shows mannose-specific adhesion, to significantly enhance the mannose-specific adhesion phenotype of a cell.

Variants of SEQ ID NO: 2 include nucleic acid sequences which hybridise to SEQ ID NO: 2, or variants of SEQ ID NO: 2 as described above, under stringent hybridisation conditions and encode a protein which retains the ability to bind mannose specifically, i.e. to confer a mannose-specific adhesion phenotype to the host cell in which it is expressed or, if the host cell already shows mannose-specific adhesion, to significantly enhance the mannose-specific adhesion phenotype of a cell.

Specific binding to mannose refers to the ability of the protein (or variant) to confer a mannose-specific adhesion phenotype to a cell in which the protein is expressed or, if the host cell already shows mannose-specific adhesion, to significantly enhance the mannose-specific adhesion phenotype of a cell. This can be tested by various tests known in the art. One suitable test for determining whether the ability to bind mannose is retained by a variant is the overexpression of the nucleic acid sequence encoding the variant in a suitable host cell, such as a knock-out mutant of *L. plantarum,* which comprises no functional mannose-specific adhesin (SEQ ID NO: 1), followed by a test suitable for determining mannose-specific adhesion of the recombinant host cell (such as the yeast agglutination test, etc., as described elsewhere herein).

For expression in eukaryotic cells it may de desirable to modify one or more amino acids at the N-terminal of the protein, in order to create an optimal translation initiation context in eukaryotic cells.

### Vectors according to the invention

In one embodiment, the invention provides a (expression) vector comprising at least one nucleic acid sequence encoding the mannose-specific adhesin, or a functional variant thereof as described above. Thus, the nucleic acid molecule encoding mannose-specific adhesin, or a functional variant thereof, is operatively linked to at least one regulatory DNA element allowing the expression of said nucleic acid in a prokaryotic or a eukaryotic cell. Usually the nucleic acid vectors of the invention will consist of DNA. These nucleic acid vectors may be provided using techniques known per se, for which reference is made to standard handbooks such as e.g. Sambrook *et al.* (1989) and Sambrook and Russell (2001).

The nucleic acid sequence encoding the mannose-specific adhesion, or functional variant thereof, is operably linked to the nucleic acid molecules required for transcription and translation of the mannose-specific adhesin in a suitable host cell, such as a (homologous or heterologous) transcription regulatory sequence upstream (5') of the coding sequence and (optionally) a 3' non-translated region. Suitable 3'nontranslated region (comprising a transcription terminator) may be those naturally associated with the coding sequence or may be identified as described by Ermolaeva et al. (J. Mol. Biol. 2000, 301: 27-33.).

Other nucleic acid elements needed for efficient transcription or translation, or suitable to enhance transcription and/or translation, may be operably linked.

Optionally, the putative N-terminal secretion signal sequence of amino acids 1-45 of SEQ ID NO: 1 may be replaced by a different signal sequence or may be removed completely. Alternatively, the mannose-specific adhesin may be operably linked to its own, natural transcription regulatory sequence and 3'non-translated sequence, that is to say that the complete gene, as naturally present in *L. plantarum,* may be incorporated in the vector. As the complete genome sequence is available, the upstream and downstream genomic sequences of the mannose-specific adhesin coding sequence are also available to the skilled person and can be used *per se* or be used to isolate or identify similar sequences.

The choice of vector elements, such as for example the choice of the transcription regulatory sequence, vector backbone, selectable marker encoding sequences, origin of replication, enhancer elements, etc., depends on the host cell in which transcription and translation are to be achieved and is easily determined by the skilled person. In principle, any transcription regulatory element that is active in the host cell may be used, and it may be homologous or heterologous to the host cell. For efficient transcription in prokaryotic cells, such as gram-positive bacteria, preferably prokaryotic transcription regulatory sequences should be used, while for transcription and translation in eukaryotic host cells preferably elements of eukaryotic origin are used. In one embodiment preferably a promoter which is homologous to the host cell is used. For example, *L. plantarum* transcription regulatory sequences which are either constitutively active or which are inducible, e.g. induced in the gastrointestinal tract of a subject (or in a specific part of the gastrointestinal tract), may be used. Such regulatory sequences are available in the art (Kleerebezem *et al.,* supra). For expression of the mannose-specific adhesin or any variant thereof in a bacterial host cell in general nucleic acid sequences from food-grade bacteria, such as lactic acid bacteria, are preferred. Strong constitutive promoters and promoters which are strongly induced following induction are especially preferred.

Other suitable constitutive promoters are the *usp45* promoter (van Asseldonk et al., 1990, Gene, 95: 155-160), the *nisR* promoter (de Ruyter et al., 1997, J. Bacteriol., 178: 3434-3439), the *pepN* promoter (Tan et al., 1992, FEBS Lett., 306: 9-16) and the promoters mentioned in de Vos and Simons in "Genetics and Biotechnology of Lactic Acid Bacteria", Gasson and de Vos, eds., pp. 52-106, Chapman and Hall, 1994. Examples of suitable regulated promoters are the bacteriophage *31* middle promoter and ori-based expression system (O'Sullivan et al., 1996, Biotechnology, 14: 82-87), the *xylA* promoter (Lokman et al., 1994, Mol. Gen. Genet., 245: 117-125), the repressor/operator system of bacteriophage *rlt* (Nauta et al., 1996, Mol. Microbiol., 19: 1331-1341), the acid-inducible or acid-responsive F₁ F₀-ATPase promoter of *L. acidophilus* (U.S. Patent No. 6,242,194), the promoters of the lactose operon of *S*. *thermophilus* and *lac ABCDFEGX* operon of *L*. *lactis* (see, e.g., Simons et al., 1993, J. Bact. 175:5186-5175; Mollet et al., 1993, J. Bact. 175:4315-4324), and the salt-inducible promoters of *L. lactis* that are disclosed in U.S. Patent No. 6,140,078.

Another suitable class of promoters are the auto-inducible promoters as described in e.g. Kuipers et al. (1997, Tibtech, 15: 135-140). Non-limiting examples are the promoters from the bacteriocin gene cluster of *Carnobacterium piscicola,* the Sakaricin gene cluster from *L. sake,* as well as (presumably) the Subtilin gene cluster from *Bacillus subtilis.* According to the invention these also include promoters for similar, non-bactericidal signal proteins involved in the quorum sensing process in microorganisms, such as the promoter for Plantaricin A from the bacteriocin gene cluster of *L. plantarum.* Reference is made to Kleerebezem et al. (1997, Mol. Microbiol. 24: 895-904) and the references mentioned therein. Suitable promoters used are the auto-inducible promoters from the nisin gene cluster of *L. lactis,* especially the *nisA* and *nisF* promoters, as described in the EP 0 712 935 *(nis A)* and also in De Ruyter et al. (1996, Appl. Environ. Microbiol. 62: 3662-3667; and Kuipers et al., *supra* and the references given therein). These promoters have already been used for the controlled (over-) expression of homologous and heterologous polypeptides (such as the *gusA* reporter gene from *E. coli, pepN,* and the lytic genes *lytH* and *lytA)* in lactic acid bacteria and for the nisin-induced expression in heterologous hosts such as *L. helveticus* and *Leuconostoc lactis.* Further suitable promoters for use in the nucleic acid molecules of the invention are reviewed by de Vos (1999, Int. Dairy J. 9:3-10; 1999, Curr. Opin. Microbiol. 2:289-295).

The vector may further comprise one or more genes encoding selectable markers, such as genes encoding antibiotic resistance, heavy metal resistance or the ability to utilise certain sugars. As in one embodiment host cells comprising the vectors are ingested by human subjects, all genetic elements used are preferably food grade, such as sugar utilisation genes as markers or food grade auxotrophic markers. Examples of sugar utilisation genes are *xylRAB* from *Lb. pentosus* (Posno et al., 1991, Appl. Environ. Microbiol. 57: 2764-2766), *levA* from *Lb. plantarum* (Wanker et al., 1994, Appl. Environ. Microbiol. 60: 1401-1413) or *Lb. casei* (Wanker et al., 1995, Appl. Microbiol. Biotechnol. 43: 297-303) and *scrA*/*scrB* from *P. pentosaceus* (Leenhouts et al., 1998, Appl. Microbiol. Biotechnol. 49: 417-423). Examples of suitable food grade auxotrophic markers are tRNA (gln) of *L. lactis* (Dickely et al., 1995, Mol. Microbiol. 15: 839-847) and *alr of L. lactis* or *Lb plantarum* (Bron et al., 2002, Appl. Environ. Microbiol. 68: 5663-5670).

Likewise, the vector may comprise a nucleic acid sequence encoding a sortase enzyme, preferably *srtA* of *L. plantarum,* which is co-transferred and co-expressed in the host cell. If the host cell already produces a functional sortase enzyme, i.e. a sortase enzyme which enables correct biogenesis of the mannose-specific adhesin of SEQ ID NO: 2 or a variant thereof, it is not necessary to co-express a sortase enzyme in the host. For example for expression in other lactic acid bacteria or other gram-positive bacteria most likely the introduction of mannose-specific adhesin alone will be sufficient to modify the phenotype of the cell as desired. However, if the host cell does not comprise a functional sortase enzyme, co-expression of a functional sortase may be required, as sortase is required for cross linking of the protein to the cell-wall of the host cell. It is irrelevant whether the sortase gene is introduced together with the mannose-specific adhesion (e.g. on one vector or on separate vectors) or is introduced independently, as long as the mannose-specific adhesion can be cross-linked to the cell wall of the host cell so that it can mediate mannose-specific adhesion of the host cell. Obviously, where the protein is to be secreted, for example for manufacturing compositions comprising the (purified) protein or for *in vivo* secretion in the gastrointestinal tract, cell wall anchorage is not desired and can be prevented by choosing a host which does not mediate cell-wall anchorage (e.g. a functional sortase deficient host cell) or by modifying or deleting the LPXTG-like motif which is part of the mannose-specific adhesin sequence. In such instances, the existing signal peptide may be replaced by a suitable secretion signal (see above).

For other uses the localisation of the protein may not be of importance. For example, if the protein is localised within the cell or within a specific cellular compartment, it may be isolated by cell lysis and optionally subsequent purification steps prior to making a suitable pharmaceutical or nutraceutical composition.

Bacteria often comprise natural plasmids, which have the ability to be transferred between bacteria by conjugal transfer. Such plasmids or derivatives or parts thereof are often utilised as cloning and expression vectors. *Lactobacillus plantarum* species often harbour several natural plasmids (Ruiz-Barba et al. 1991) of which some have been characterised and sequenced (Bates and Gilbert, 1989, Danielsen, 2002, Eguchi et al., 2000, Kanakeo et al., 2000, Leer et al., 1992, Skaugen, 1989, Vujcic and Topisirovic, 1993). It is understood that the use of such plasmids or vectors derived therefrom may also be used as vectors for expressing the mannose-specific adhesin (or variant thereof) in a host cell according to the invention.

Also fusion proteins comprising the mannose-specific adhesin, or a fragment or variant thereof which retains the ability to bind mannose, linked in frame to another coding sequence (or part thereof) is an embodiment of the invention. Fusion proteins comprising various functional domains can be generated by in frame fusion of the nucleic acid sequences encoding the domains, so that upon transcription and translation in a host cell a fusion protein is generated. In this way, different functional domains can be combined in one protein, which may be advantageous. For example, domains of biologically active proteins (or whole proteins), such as proteins with known therapeutic properties, may be added so that these are displayed on the cell surface of the host cell.

In another embodiment one or more biologically active proteins and the mannose-specific adhesin according to the invention are co-expressed in a single host cell, so that the host cell may be used as a vehicle to deliver these proteins to the location where the host cell binds, for example the human intestine. Co-expression may be achieved by various means, for example co-transformation of several vectors or a single vector comprising two genes, or the vector may comprise one or more nucleotide sequences encoding different (or identical) proteins under control of a single regulatory sequence, providing bicistronic or multicistronic transcripts. The biologically active protein(s) may in this way be secreted by the host cell, while the mannose-specific adhesin binds the host cell to receptors found on the epithelial cells of a subject.

It is understood that the nucleic acid constructs or vectors of the invention may further comprise additional sequence elements for various functions known in the art *per se.* Such sequence element include e.g.: autonomously replicating sequences or origins of replication for extrachromosomal multiplication and maintenance of the vector; sequences for promoting integration of the vector or chimeric gene into the host's genome; sequences for homologous recombination; sequences for site-specific integration and/or recombination; selection marker genes; reporter or indicator genes; sequences for promoting conjugation or other means of transfer of genetic material etc.

### Host cells according to the invention

In another aspect of the invention a host cell comprising a nucleic acid sequence encoding a mannose-specific adhesin or variant thereof as described above is provided, or a vector as described above. The expression of the nucleic acid sequence in the host cells confers either mannose-specific adhesion to the host cell, in cases where the host cell lacks this phenotype, or enhances mannose-specific adhesion of the host cell, in cases where the host cell already shows the ability to adhere in a mannose-specific manner. As mentioned, the nucleic acid sequence encoding the mannose-specific adhesin need not be integrated into the genome of the host cell, as long as the phenotype conferred by the expression of the nucleic acid sequence remains stable.

It also does not matter how the mannose-specific adhesin encoding sequence is introduced into the host cell, as long as the phenotype of the host cell is modified in the desired way and remains stable during subsequent multiplication of the host cell. There are, therefore, various methods available in the art for introducing the nucleic acid sequences or vector(s) encoding the mannose-specific adhesin or variants thereof into a host cell, such as transformation of cells or conjugative transfer between bacteria. Methods for transforming bacterial cells are for example described in "Genetics and Biotechnology of Lactic Acid Bacteria", Gasson and de Vos, eds., Chapman and Hall, 1994. Similarly, methods for introducing recombinant DNA into fungi, plants and animals are well known, such as particle gun transformation, protoplast transformation, electroporation, *Agrobacterium* mediated transformation and the like.

In case a host of the invention is constructed through genetic engineering such that the resulting recombinant host comprises only sequences derived from the same species as the host is, albeit in recombined form, the host is said to be obtained through "self-cloning". Hosts obtained through self-cloning have the advantage that there application in food (or pharmaceuticals) is more readily accepted by the public and regulatory authorities as compared hosts comprising foreign (i.e. heterologous) nucleic acid sequences. The present invention thus allows the construction of self-cloned L. *plantarum* and other lactobacillus hosts for food, pharmaceutical or nutraceutical applications (see also de Vos, 1999, Int. Dairy J. 9: 3-10) and such self-cloned hosts are one further aspect of the invention.

The host cell may in principle be any prokaryotic or eukaryotic cell, such as human or animal cell lines, gram positive or gram negative bacteria, fungi, viruses, or other microorganisms. In one preferred embodiment the host cell is a bacterial cell, such as a gram positive bacterium, preferably a food-grade bacterium. A host or host cells may also be a mutant, generated by classical (X-ray or chemical) mutagenesis or the host may already have been transformed previously. Other hosts are food-grade fungi and yeasts, such as bakers or brewers yeast, such as species of the genus Saccharomyces, Pichia, or Hansenula. In a preferred embodiment the host cell is a probiotic bacterium, which in itself has a beneficial effect when ingested by a subject.

The host cell may be a gram-positive bacterium that belongs to a genus selected from the group consisting of *Lactobacillus, Lactococcus, Leuconostoc, Carnobacterium, Bifidobacterium, Bacillus, Streptococcus.* More preferably, the host cell is selected from a Gram-positive bacterium that belongs to a species selected from the group consisting of *L. acidophilus, L. amylovorus, L. bavaricus, L. brevis, L. caseii, L. crispatus, L. curvatus, L. delbrueckii, L. delbrueckii* subsp. *bulgaricus, L. fermentum, L. gallinarum, L. gasseri, L. helveticus, L. jensenii, L. johnsonii, L. minutis, , L. murinus L. paracasei, L. plantarum, L. pontis, L. reuteri, L. sacei, L. salivarius, L. sanfrancisco, Lactobacillus ssp., Pediococcus spp., Camobacterium spp. C. piscicola, B. subtilis, Leuconostoc mesenteroides, Leuconoctoc lactis, Leuconostoc ssp, B. bifidum, B. longum, B. infantis, B. breve, B. adolescente, B. animalis, B. gallinarum, B. magnum,* and *B. thermophilum.*

As variants of the nucleic acid sequence may be obtained from any other organism, the nucleotide sequence encoding the mannose-specific adhesion may be homologous or heterologous to the host cell. In one embodiment the host cell originally lacks the ability to bind mannose specifically and the transfer of one or more copies of the adhesin according to confers this novel phenotype onto the host cell. The modified host cell thereby has acquired a number of novel, testable properties, namely it can bind mannose (e.g. mannose coated agarose beads), it can bind to cells comprising mannose (e.g. oligomannose chains exposed on the cell surface), e.g. human colonic cell lines and it can agglutinate mannose comprising cells, such as yeast cells or erythrocytes in a mannose-specific manner. A positive score in these tests indicates that the adhesin protein is functionally exposed on the outside of the modified cell. This is an indication that the cell also has novel properties which are useful for a variety of pharmaceutical and/or nutraceutical uses.

In another embodiment, the host cell already possesses the ability to bind mannose. For example the wild type *L. plantarum* strain WCFS1 already has this ability, as demonstrated in the tests used. However, it was surprisingly found that the introduction of at least one further copy of the adhesin coding sequence results in a significant improvement of the strain's of mannose-specific adhesion. It is thus possible to significantly enhance the phenotype by introducing additional functional copies of the mannose-specific adhesin into host cells. Such modified host cells have thus improved properties compared to the unmodified cells, as can be determined using the same test, as further described below. A host cell comprising one or more additional copies of the gene can be easily distinguished from an unmodified host cell using common molecular methods, such as PCR, hybridisation, molecular markers, etc. Also quantitative reverse transcriptase PCR (RT-PCR) or Northern blots may indicate the mRNA abundance. It is presumed that the additional copy or copies enhance the phenotype by displaying more mannose-specific adhesin proteins on the cell surface. Hence, a similar effect may be expected when the expression of a single copy is increased. Quantitative RT-PCR may therefore be used to quantify transcript levels and to select host cells which produce more mRNA transcripts of the mannose-specific adhesin (or variant) gene. In this way existing strain collections may be screened for strains with increased transcript levels, which may as a result show an enhanced mannose-specific adhesion phenotype. This may also be done following for example mutation of strains or following transformation as described above or conjugation between strains, whereby a copy of the mannose-specific adhesion encoding gene may be transferred from one strain to another. Especially strains which produce at least 10%, 20%, 30%, preferably at least 50, 70, 90 or 100% more mannose-specific adhesin mRNA transcript (or of a variant thereof) and which additionally show a newly acquired or significantly improved mannose-specific adhesion phenotype in at least the yeast agglutination test are an embodiment of the invention.

Following introduction of at least one sequence encoding a mannose-specific adhesin according to the invention the hosts or host cells (1) express, or are able to express (e.g. upon induction), the mannose-specific adhesion sequence according to the invention, (2) expression either confers or enhances the mannose-specific adhesion properties as testable in the test described. Preferably, the cell is capable of surviving passage through a subject's gastrointestinal tract and preferably has an improved capability of adhering to and colonizing a mucosal surface lining a subjects gastrointestinal tract compared to the non-modified cell. Further, administration of the cells or compositions comprising these preferably, reduces, prevents or delays infections caused by pathogens, especially bacterial pathogens which are members of the Enterobacteriaceae, such as e.g. *E. coli, Eneterobacter, Klebsiella, Proteus.* In particular the cells or compositions of the invention reduce, prevent or delay infections caused by pathogenic bacteria comprising type 1 fimbriae, such as e.g. *Salmonella, Closteridium, Campylobacter* and *Streptococcus.*

It is in this respect irrelevant by which precise mechanism pathogen infection is prevented, reduced or delayed following administration of the recombinant host cells according to the invention. The administered cells of the invention may for example compete for mannose-containing binding-sites with the pathogen or they may compete fro growth limiting substrates, or lead to a decrease in translocation of pathogenic or potentially pathogenic bacteria over the intact epithelial surface, it may inhibit or reduce adherence of pathogenic bacteria to the epithelial cell surface, it may influence the immune system or decrease inflammatory damage, or any combination thereof, etc.

It is understood that any derivatives of the (modified) host cells described herein, which retain the desired phenotype, such as obtained through bacterial multiplication/growth are also encompassed herein.

### Functional tests according to the invention

There are a number of known test which are suitable for determining whether a cell, especially a bacterial cell, displays mannose-specific adhesion properties. All test measure mannose binding. The use of one or more of these test will allow to determine whether a cell which has been modified by introducing at least one mannose-specific adhesin encoding nucleic acid sequence according to the invention has acquired a mannose-specific adhesion phenotype (while lacking this phenotype before) or has acquired a significantly enhanced mannose-specific adhesion phenotype compared to the phenotype displayed prior to modification.

A cell is defined to display mannose-specific adhesion properties, i.e. has the ability to bind mannose, if it agglutinates yeast cells in the yeast agglutination test (see below) at the highest bacterial cell density at which agglutination can be detected by light microscopy. Preferably agglutination of yeast cells occurs in a mannose-specific manner as may be established by inhibition of agglutination by e.g. methyl-α-D-mannoside (see below). Maximal cell densities at which yeast agglutination can still be assessed using light microscopy are approximately 6* 10⁹ colony forming units per ml in the final agglutination assay (see below). In analogy, yeast agglutination efficiency can be quantitatively expressed as the mean agglutination titre and standard deviation, which can also be applied to other materials, like purified mannose adhesion protein or (sub)cellular fractions of mannose adhesion producing cells.

An "enhanced mannose-specific adhesion" phenotype refers to an improvement of mannose-specific adhesion of at least 20%, 30%, 40% or 50% or more of the agglutination titre in the yeast-agglutination test.

### Yeast agglutination test

The "Yeast agglutination test" is a test suitable for testing mannose-specific adhesion of cells, especially microorganisms. The cell-wall of *Saccharomyces cerevisiae* comprises mannose containing polysaccharides (mannans). In this test *S. cervisiae* cells are contacted with cells to be tested (especially bacteria), at various concentrations, in the presence or in the absence of a selected monosaccharide, one of which is mannose. Bacterial strains are grown overnight in appropriate media, washed and suspended in 0.1 culture volume PBS (pH 7,4). The cell number was recorded by plating out tenfold dilutions of the bacterial suspensions on appropriate agar plates and determining CFU/ml after 48 hours growth at 37°C. 50 µl of twofold dilutions of the bacterial suspensions in PBS were prepared in microtitre plates (96-well u-shaped, Greiner bio-one, Alphen a/d Rijn, The Netherlands). 50 µl PBS (pH 7.4) or PBS with methyl-α-D-mannopyranoside (final concentration: 25 mM; Sigma-Aldrich Chemie BV, Zwijndrecht, The Netherlands) were added as well as 100 µl of 1 % (w/v) *Saccharomyces cerevisiae* suspended in PBS after growth in malt extract medium (Oxoid, Haarlem, The Netherlands). The plate was shaken for 10 minutes at room temperature and volumes of 50 µl were taken from each well to examine agglutination by bright-light microscopy (200-fold magnification, inverted microscope Nikon Eclipse TS100). The reciprocal of the highest dilution that still resulted in visible agglutination was recorded as the agglutination titre. Mean agglutination titre and standard deviation of three independent assays were calculated. This procedure is a slight adaptation of the method previously described on pages 2244 and 2245 of Adlerberth *et al.,* supra). Erythrocyte agglutination test (Hemagglutination test)

The glycoproteins of erythrocytes comprise mannose and it is known that certain *E. coli* and some *L. plantarum* strains agglutinate erythrocytes in a mannose-sensitive manner. This test involves mixing various dilutions of cells to be tested with an equal volume erythrocyte suspensions, either in a normal buffer solution or in a solution supplemented with D-mannose or methyl-D-mannoside. After about 15 minutes agglutination is assessed visually, using for example a light microscope at a x250 magnification. The reciprocal of the highest dilution of cell suspension which results in visible agglutination within the incubation period is recorded as the agglutination titre. If agglutination of cells and erythrocytes is significantly reduced or completely inhibited by the presence of D-mannose or methyl-α-D-mannoside, the cells exhibit a mannose-specific adhesion phenotype. Details of this test are found for example in Adlerberth et al. (supra).

### Direct mannose binding test

In this test D-mannose coated agarose beads and non-coated beads are mixed with the cells to be tested and binding is measured microscopically at about 500x magnification. If the cells adhere to the D-mannose coated beads but not to the non-coated beads the test is positive and the cells show a mannose-specific adherence phenotype. Obviously, mannose may be immobilised by other means and adherence may be assessed in other ways. Again, details can be found in Adlerberth *et al.,* supra.

### Human cell binding test

A suitable cell line, which is known to comprise mannose moieties on its surface, such as human adenocarcinoma cell line HT-29, is mixed with the cells to be tested, either in the presence or absence of selected monosaccharides. The number of cells attached to at least 40 HT-29 cells is determined using interference-contrast microscopy at a magnification of about x500. The specific ability of mannose to significantly reduce adherence of the test cells to HT-29 cells shows that the test cells exhibit a mannose-specific adherence phenotype. In particular, other monosaccharides such as D-glucose, methyl-α-D-glucoside, L-fucose, N-acetyl-glucosamine, galactose, etc. are not able to inhibit or reduce adherence. *L. plantarum* strain 299v has been described to adhere to HT-29 cells at about 10 bacteria per cell in the absence of mannose. In the presence of methyl-α-D mannoside adherence was reduced by 45-73%.

These above tests are non-limiting, and alternative or modified tests can be easily devised by a skilled person.

### Screening methods according to the invention

In a further aspect the invention relates to method, in particular screening methods, for determining whether a bacterium has probiotic properties, is capable of adhering to human cells and/or has the ability to bind mannose. The method preferably comprises determining the presence or absence of a nucleotide sequence comprised in SEQ ID NO: 2 or parts or variants. Preferably the presence of the nucleotide sequence, or more preferably its expression is indicative of probiotic properties. The bacterium preferably is a bacterial strain or (single colony) isolate from a genus selected from the group consisting of *Lactobacillus, Lactococcus, Leuconostoc, Camobacterium, Bifidobacterium, Bacillus, Streptococcus.*

In one embodiment of the invention PCR primers and probes, as well as kits comprising these, for detecting the mannose-specific adhisin DNA and/or RNA sequences are provided. PCR primers which pair to amplify SEQ ID NO: 2 or parts or variants thereof can be synthesised based on SEQ ID NO: 2 as known in the art (see Dieffenbach and Dveksler 1995, PCR Primer: A laboratory manual, Cold Spring Harbor Lab. Press, or McPherson et al. 2000, PCR-Basics: From background to bench, first edition, Springer Verlag, Germany). PCR primers may, for example, comprise at least 18, 19, 20 or more nucleotides identical to part of SEQ ID NO: 2 (or to its complementary strand), or they may be degenerate and not match 100% to SEQ ID NO: 2 (or its complementary strand).

Likewise, SEQ ID NO: 2, or fragments thereof, can be used as hybridisation probe. A detection kit may comprise either mannose-specific adhesin primers or probes, an associated protocol to use these and optionally other reagents required.

The primers, probes or kits may be used to a) quickly identify cells which comprise one or more nucleic acid sequence according to the invention and may as a result have a mannose-specific adhesion phenotype or b) isolate variants of the nucleic acid sequence encoding the mannose-specific adhesin according to the invention from suitable cells.

The screening method for determining whether one or more cells, especially bacteria, are capable of mannose-specific adherence involves the steps of a) determining the presence or absence, within the DNA or RNA of the cell, of at least one nucleotide sequence according to the invention, b) selecting the cell which comprises the nucleotide sequence(s) and c) optionally testing the mannose-specific phenotype of the cell using for example the yeast agglutination test described.

Step a) may for example involve PCR amplification or nucleic acid hybridisation, or fingerprinting methods, such as AFLP, RFLP, RAPD, or other methods, as known in the art. Generally, the cells are grown and harvested, DNA or RNA is isolated from them and the specific DNA or RNA is detected using direct or indirect detection methods as known. The cells screened are preferably bacteria, especially Gram-positive bacteria that belong to a genus selected from the group consisting of *Lactobacillus, Lactococcus, Leuconostoc, Carnobacterium, Bifidobacterium, Bacillus, Streptococcus.*

The screening method may also make use of protein detection methods, wherein for example bacterial collections are screened for the presence / absence of the mannose-specific adhesin protein, or variants thereof. The protein may be detected directly or indirectly, using for example immunihostochemical methods, Enzyme-linked immunosorbent assay (ELISA) or Western blotting, and other methods known in the art. The assays may use a wide variety of labels, such as radioisotopes, enzymes, fluorescers, chemiluminescers, spin labels, and the like. Antibodies (e.g. monoclonal antibodies) for use in such detection assays may be raised against the mannose-specific adhesin (or a variant thereof) using known methods.

In a preferred embodiment, the expression of the nucleic acid molecule encoding the mannose-specific adhesin according to the invention confers to the host cell, or significantly improves, the ability to adhere to human cells *in vitro* (e.g. cultured cell lines) and/or is *vivo,* especially to cells found in the human gastrointestinal tract, such as epithelial cells (e.g. intestinal epithelial cells, vaginal epithelial cells, urethral epithelial cells, etc.). Especially attachment and colonisation of the cell to mucosal surface are preferably improved. Also persistence within the subject is preferably longer.

The ability to adhere can, for example, be tested using the human cell binding test above. In this test the adherence of pathogen cells (e.g. pathogenic *E. coli, Salmonella typhimurium,* etc.), which are known to bind to the cells in a mannose-sensitive manner, may be compared to the adherence of the recombinant host cells and to mixtures of the pathogen cells and the recombinant host cells. Preferably, addition of the recombinant host cells to the pathogen cells will significantly reduce adherence of the pathogen cells. Especially addition of the recombinant host cells prior to the addition of the pathogen may have a significant effect on reducing pathogen adherence.

In vivo test may be carried out in test animals, such as rats, mice, rabbits and the like. In such test compositions comprising the recombinant host cells are administered to the subject's gastrointestinal tract (e.g. orally or rectally) for several days (while not being administered to a control group), followed or concomitantly with administration of a pathogenic bacterium. Tissue samples may then be taken from the gastrointestinal tract are analysed to determine whether the prior or concomitant administration of the host cell has decreased attachment and colonisation by the pathogen.

### Compositions according to the invention and their use

### a) host cell comprising compositions and methods

In a further embodiment compositions (pharmaceutical and/or nutraceutical compositions and/or foods and/or food ingredients and/or probiotics) are provided which comprise the host cells as described above and one or more pharmaceutically or physiologically acceptable carriers. How such compositions are made is generally known in the art.

Additionally, a method for producing the pharmaceutical and/or nutraceutical composition comprising a host cell, said host cell having been modified to comprising at least one nucleic acid sequences encoding a mannose specific-adhesin' of SEQ ID NO: 1, or a functional variant thereof, whereby the host cell has an acquired a mannose-specific adhesion phenotype or has an enhanced mannose-specific adhesion phenotype compared to the unmodified host cell, the method comprising the steps of a) culturing the host cell in a suitable medium and b) mixing the host cell with one or more pharmaceutically (physiologically) acceptable carriers. It is clear that the host cells may be purified from the culture medium prior to mixing these with additional components.

Pharmaceutical/nutraceutical compositions will usually be used for enteral, for example oral, nasal/inhalation, vaginal or rectal administration or tube feeding. Pharmaceutical compositions will usually comprise a pharmaceutical/physiologically acceptable carrier in addition to an effective amount of the host cells of the invention. An "effective amount" refers to the minimal dosage of host cells which is able to significantly reduce or prevent infection of at least one species of pathogenic microorganisms, such as an enteropathogenic bacterium comprising type 1 fimbriae. The effective amount can easily be determined by a skilled person. The preferred form of the composition depends on the intended mode of administration and therapeutic, prophylactic or health-beneficial application. The carrier can be any compatible, nontoxic substance suitable to deliver the host cells to the desired body cavity, e.g. the intestine of a subject. E.g. sterile water, or inert solids may be used as the carrier usually complemented with pharmaceutically acceptable adjuvants, buffering agents, dispersing agents, stabilisers and the like. The compositions may further comprise additional biologically or pharmaceutically/physiologically active ingredients, such as additional microorganisms (e.g. additional probiotic bacteria), biologically active proteins, etc..

The concentration of host cells will vary, depending on the formulation and application of the composition. Preferably, the compositions will contain the host cells in amounts that allow for convenient (oral) administration of the host cells, e.g. as or in one or more doses per day or per week. In particular, the preparations may contain a unit dose of the host cells. For enteral application the dose of host cells is preferably at least 1x10⁶cfu, preferably between about 1x10⁶ - 1x10¹² cfu (colony forming units) per day, more preferably between about 1x10⁷ - 1x10¹¹ cfu/day, more preferably about 1x10⁸ - 5x10¹⁰ cfu/day, most preferably between 1x10⁹-2x10¹⁰ cfu/day and can be fine-tuned empirically, without undue experimentation. The host cells are preferably live or viable cells (e.g. lyophilised cells). The effective dose may be subdivided into several smaller dosages and administered for example in two, three or more portions per day. The cells may be protected by a protective coating, in order to increase survival during passage through the stomach.

In a further embodiment dead or non-viable bacterial cells of the strain(s) are used in the above compositions, instead of or in addition to live (or viable) bacteria, as for example described in WO01/95741. The amount of dead or non-viable cells used may, for example, be equivalent to that used for live bacteria. Suitable amounts can be easily determined by a skilled person. In such compositions, the amounts of cells are counted (e.g. using a flowcytometer) or measured in a different way as known to a skilled person, as measurement as 'colony forming units' is not feasible.

The host cells may also be present in the composition in the form of viable, lyophilised (freeze dried) cells, which become active again after administration or reconstitution with liquid.

The compositions will either be in liquid, e.g. a stabilised suspension of the host cells, or in solid forms, e.g. a powder, or in semi-solid form. E.g. for oral administration, the host cells can be administered in solid dosage forms, such as capsules, tablets, and powders, or in liquid dosage forms, such as elixirs, syrups, and suspensions. The host cells can be encapsulated in gelatin capsules together with inactive ingredients and powdered carriers, such as e.g. glucose, lactose, sucrose, mannitol, starch, cellulose or cellulose derivatives, magnesium stearate, stearic acid, sodium saccharin, talcum, magnesium carbonate and the like.

In one embodiment a composition according to the invention is suitable for consumption by a subject, preferably a human or an animal. Such compositions may be in the form of a food supplement or a food or food composition, which besides the host cells of the invention also contains a suitable food base. A food or food composition is herein understood to include liquids for human or animal consumption, i.e. a drink or beverage. The food or food composition may be a solid, semi-solid and/or liquid food or food composition, and in particular may be a dairy product, such as a fermented dairy product, including but not limited to yoghurt, a yoghurt-based drink or buttermilk. Such foods or food compositions may be prepared in a manner known per se, e.g. by adding the host cells of the invention to a suitable food or food base, in a suitable amount. In a further preferred embodiment, the host cells are micro-organisms that are used in or for the preparation of a food or food composition, e.g. by fermentation. Examples of such micro-organisms include baker's or brewer's yeast and lactic acid bacteria, such as probiotic lactic acid strains. In doing so, the host cells of the invention may be used in a manner known per se for the preparation of such fermented foods or food compositions, e.g. in a manner known per se for the preparation of fermented dairy products using lactic acid bacteria. In such methods, the host cells of the invention may be used in addition to the micro-organism usually used, and/or may replace one or more or part of the micro-organism usually used. For example, in the preparation of fermented dairy products such as yoghurt or yoghurt-based drinks, a food grade lactic acid bacterium of the invention may be added to or used as part of a starter culture or may be suitably added during such a fermentation.

In another aspect the invention relates to a method for (site-specific) production of a polypeptide or a biologically active substance at a mucosal surface of a subject. The method comprises the step of administering to the subject a composition comprising the host cells as described above. As the host cells are particularly suited to attach to and colonise mucosal surfaces, they can be used effectively to deliver other biologically active substances to the mucosal surface of subject. This can be achieved by either pre-loading the cells with such substances, or by co-expressing biologically active proteins or polypeptides in such cells, which are then produced and/or released locally at the site of host-cell colonisation. These may be membrane bound or secreted proteins or protein fragments with biological activity. In one embodiment, the polypeptide is an antigen from a human or animal pathogen. The *in situ* production of the pathogen's antigen(s) allows for the vaccination of the human or animal to which the composition is administered. In another embodiment the polypeptide or the biologically active substance is a health-promoting factor.

### b) protein comprising compositions and methods

In another embodiment of the invention a pharmaceutical and/or nutraceutical composition comprising a mannose-specific adhesin, or a variant thereof, as described, and one or more pharmaceutically (physiologically) acceptable carriers is provided. In another embodiment a method for producing a polypeptide according to the invention is provided, the method comprising the steps of:
a) culturing a host cell which has been modified to comprise at least one (newly introduced or additional) nucleic acid sequence encoding a mannose-specific adhesin, or a variant thereof, under condition conducive to the expression of the polypeptide; and, b) recovery of the polypeptide.

Also provided is a method for producing the pharmaceutical (and/or nutraceutical) composition, the method comprising the steps a) and b) here above and further comprising the step of mixing the polypeptide with a pharmaceutically (physiologically) acceptable carrier. Prior to mixing the polypeptide with the carriers, it may optionally be purified using protein purification methods known in the art. Preferably, the polypeptide is secreted from the cultured host cell and can be easily recovered from the culture medium.

The embodiments described above for compositions comprising host cells apply analogously to compositions comprising the mannose-specific adhesin or variant(s) thereof.

### Use of the compositions

Provided is also a method for treating or preventing a pathogen infection of the gastrointestinal tract, the urinary tract, and/or the vagina comprising administering to a patient in need of such treatment an effective amount of a composition comprising host cells as described above or comprising a suitable amount of the mannose-specific adhesin, as described. Especially, infection of a mucosal surface in a subject caused by a pathogenic bacterium which expresses type 1 fimbriae can be prevented or significantly reduced by administration of a composition according to the invention.

The host cells or mannose-specific adhesin polypeptide as described can, thus, be used for the manufacture of a medicament for the treatment or prevention (prophylactic treatment) of a pathogen infection of the gastrointestinal tract, the urinary tract, and/or the vagina, in particular for the treatment or prevention of infections of a mucosal surface caused by one or more bacterial species which express type 1 fimbriae.

In particular, the compositions may be used for the treatment, prevention or delay of infections caused by the pathogenic microorganisms as indicated above.

The following non-limiting Examples describe the identification and isolation of mannose-specific adhesin. Unless stated otherwise, the practice of the invention will employ standard conventional methods of molecular biology, virology, microbiology or biochemistry. Such techniques are described in Sambrook et al. (1989) Molecular Cloning, A Laboratory Manual (2nd edition), Cold Spring Harbor Laboratory, Cold Spring Harbor Laboratory Press; in Sambrook and Russell (2001) Molecular Cloning: A Laboratory Manual, Third Edition, Cold Spring Harbor Laboratory Press, NY; in Volumes 1 and 2 of Ausubel et al. (1994) Current Protocols in Molecular Biology, Current Protocols, USA; and in Volumes I and II of Brown (1998) Molecular Biology LabFax, Second Edition, Academic Press (UK); Oligonucleotide Synthesis (N. Gait editor); Nucleic Acid Hybridisation (Hames and Higgins, eds.), all incorporated herein by reference.

### Description of the figures

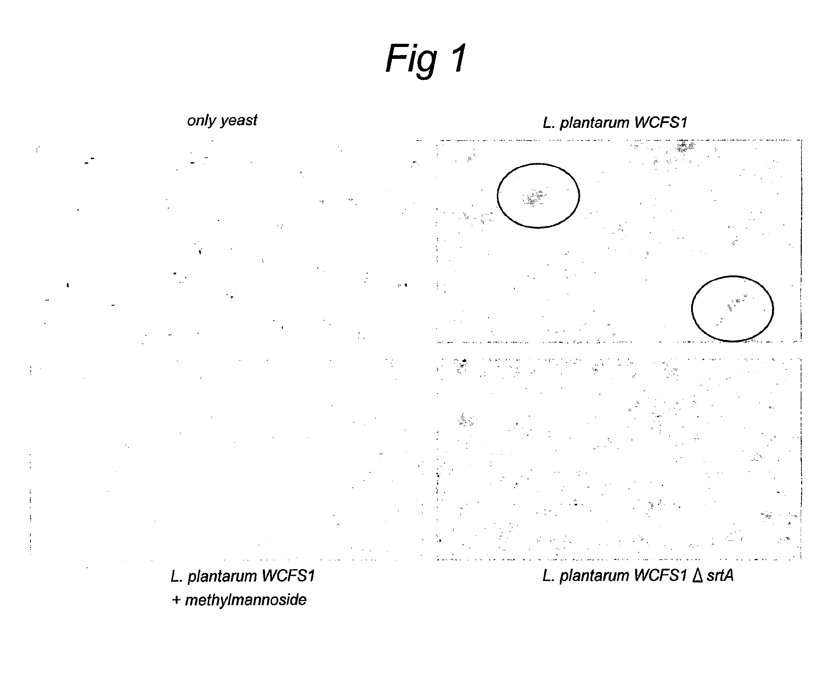

### Figure 1: Yeast mannose-specific agglutination test

Top left: only yeast; Top right: yeast cells and *L. plantarum* WCFS1; Bottom left: *L. plantarum* WCFS1 and methyl-mannoside; Bottom right: *L. plantarum* WCFS1 *ΔsrtA*

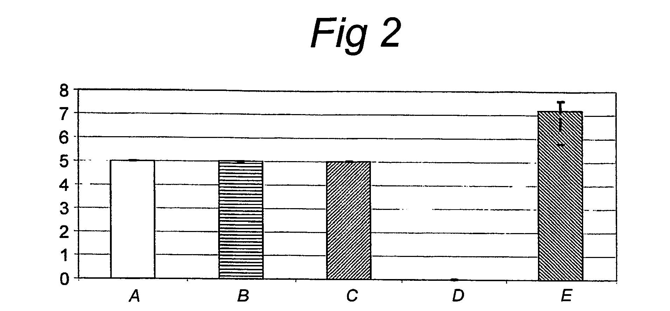

Figure 2: Agglutination of *Saccharomyces cerevisiae* mediated by *L. plantarum* WCFS1 mutant strains. Mean agglutination titres (log 2) +/- SD calculated from 3 independent experiments are displayed. Wildtype (A) and *lp_0373* mutant strains (B and C) show the same capacity to agglutinate yeast cells. In contrast, *cat*-replacement of *lp_1229* (D) leads to a complete loss of this ability, whereas overexpression of *lp_1229* (E) results in enhanced agglutination. A: *L. plantarum* WCFS1 wildtype; B: *L. plantarum* WCFS1 lp_0373::cat; C: *L. plantarum* WCFS1 lp_0373 overexpression; D: *L. plantarum* WCFS1 lp_1229::cat; E: *L. plantarum* WCFS1 lp_1229 overexpression.

### Examples

### Example 1 - in silico analysis

It was found that *in silico* analysis was not suitable to identify which gene or genes of *L. plantarum* is responsible for the mannose-specific adhesion phenotype described. To illustrate this, a list of *L. plantarum* genes which display sequence homology to genes identified in other bacterial species and which in those other species potentially play a role in adherence to mucosal surfaces is listed below.

The format is given as follows: lp_xxxx : gene name of the hit from homology search *[species of origin of the hit].* Lp_xxxx refers to the *L. plantarum* gene as designated by Kleerbezem et al. (2003) and EMBL database accession no. AL935263.

### L. plantarum gene : homology hit [species of origin of the hit]

lp_0304: aggregation promoting protein [*Lactobacillus gasseri*]
lp_0520: autoaggregation-mediating protein [*Lactobacillus reuteri*]
lp_1229: mucus binding protein precursor; Mub [*Lactobacillus reuteri*]
lp_1643: mucus binding protein precursor; Mub [*Lactobacillus reuteri*]
lp_1793: fibronectin-binding protein-like protein A [*Streptococcus gordonii*]
lp_1873: aggregation-relevant adhesin [*Streptococcus gordonii*]
lp_2278: autoaggregation-mediating *protein [Lactobacillus reuteri*]
lp_2845: aggregation promoting protein [*Lactobacillus gasseri*]
lp_2847: aggregation promoting protein [*Lactobacillus gasseri*]
lp_3050: aggregation promoting protein [*Lactobacillus gasseri*]
lp_3059: mucus binding protein precursor; Mub [*Lactobacillus reuteri*]
lp_3114: mucus binding protein precursor; Mub [*Lactobacillus reuteri*]
lp_3209: mucus adhesion promoting protein [*Lactobacillus reuteri*]
lp_3214: collagen binding protein *[*L*actobacillus reuteri*] / mucus adhesion promoting protein [*Lactobacillus reuteri*].

This list is non-exhaustive and homology may also be due to homology in only a limited, specific domain. Such regional homology in many cases does not allow function annotation and merely indicates that certain domains are conserved.

This list illustrates the impossibility of predicting which gene or genes of *L*. *plantarum* are responsible for mannose-specific adhesion, despite the availability of the complete genome sequence of *L. plantarum.*

### Example 2 - Identification of mannose specific adhesin encoding gene(s) in L. plantarum WCFS1

Mannose specific adhesion properties of *L. plantarum* strains were evaluated in a simple assay that is based on *L. plantarum* mediated yeast *(Sacharomyces cerevisiae)* agglutination, which can be inhibited by the addition of specific mannosides, as described in Adlerberth et al. (1996) with slight modifications. In brief, bacterial strains were grown overnight in appropriate media, washed and suspended in 0.1 culture volume PBS (pH 7.4). The cell number was recorded by plating out tenfold dilutions of the bacterial suspensions on appropriate agar plates and determining CFU/ml after 48 hours growth at 37°C. 50 µl of twofold dilutions of the bacterial suspensions in PBS were prepared in microtitre plates (96-well u-shaped, Greiner bio-one, Alphen a/d Rijn, The Netherlands). 50 µl PBS (pH 7.4) or PBS with methyl-α-D-mannopyranoside (final concentration: 25 mM; Sigma-Aldrich Chemie BV, Zwijndrecht, The Netherlands) were added as well as 100 µl of 1 % (w/v) *Saccharomyces cerevisiae* suspended in PBS after growth in malt extract medium (Oxoid, Haarlem, The Netherlands). The plate was shaken for 10 minutes at room temperature and volumes of 50 µl were taken from each well to examine agglutination by bright-light microscopy (200fold magnification, inverted microscope Nikon Eclipse TS100). The reciprocal of the highest dilution that still resulted in visible agglutination was recorded as the agglutination titre. Mean agglutination titre and standard deviation of three independent assays were calculated.

Initial experiments using *L. plantarum* WCFS 1 have indicated that this strain can mediate yeast-agglutination in a mannoside specific manner (Figure 1). Next to WCFS1, various *L. plantaraum* strains were tested on their capacity to agglutinate *Saccharomyces cerevisiae.* Under the applied conditions reproducible agglutination titres were obtained for individual strains. In addition, yeast cells did not agglutinate without the addition of bacteria, verifying that this process is induced by bacteria (Figure 1). Finally, it could be concluded that the yeast agglutination titres differed between the *L. plantarum* strains, showing diversity in this capacity between the strains (Table 1). Importantly, several strain lacked the capacity to agglutinate yeast cells. For all strains that displayed yeast agglutination, this agglutination could be completely inhibited by addition of methyl-α-D-mannopyranoside, demonstrating a mannose-specific mechanism (data not shown).

**Table 1: Agglutination of Saccharomyces cerevisiae mediated by various L. plantarum strains. Mean agglutination titres (log 2) +/- SD calculated from 3 independent experiments are shown. The third column provides information on the source of the L. plantarum strains.**

| ***L. plantarum* strain** | **Mean titre (log 2) ± SD** | **Source; reference** |
|---|---|---|
| ATCC 14917 | 6.0 ± 0.0 | Prickled cabbage, ATCC |
| ATCC 8014 | 0±0.0 | Maize ensilage, ATCC |
| CIP 104440 | 0 ± 0.0 | Human stool, ref. 1 |
| CIP 104441 | 0±0.0 | Human stool, ref. 1 |
| CIP 104450 | 0 ± 0.0 | Human stool, ref. 1 |
| CIP 104451 | 0 ± 0.0 | Human urine, ref. 1 |
| CIP 104452 | 7.7 ± 0.6 | Human tooth abcess, ref. 1 |
| 299 | 7.3 ± 0.6 | Human GI tract, ref. 2 |
| 299v | 6.7 ± 0.6 | Human GI tract, ref. 2 |
| NC8 | 4.0 ± 0.0 | Silage, ref. 3 |
| LM3 | 5.3 ± 0.6 | Silage, ref. 4 |
| LP80 | 6.7 ± 0.6 | Silage, ref. 5 |
| LP 85-2 | 0 ± 0,0 | Silage, NCIMB collection |
| WCFS1 | 4.8 ± 0.6 | Human saliva, sequenced strain |

| | | |
|---|---|---|
| References in Table 1: 1: Gasser and Sebald 1966, Ann Inst Pasteur (Paris) 110:261-275. 2: Adlerberth et al. AEM 3: Aukrust, T., and H. Blom. 1992. Food Res. Int. 25:253-261. 4: K. Thompson, K. McConville, L. McNeilly, C. Nicholson, and M. Collins, Abstr. 6th Symp. Lactic Acid Bacteria Genet. Metab. Appl., p. E5, 1999 5: Scheirlinck T, et al. Appl Environ Microbiol. 1989 55:2130-2137. | | |

This information was matched to the *L. plantarum* genotype-diversity database that was constructed previously on basis of *L. plantarum* strain-genotyping using the *L. plantarum* WCFS1 clone-based DNA microarrays (unpublished). This matching revealed a list of 10 *L. plantarum* WCFS1 genes of which the presence / absence score in the diversity database consistently coincides with the observed phenotypic variation observed in the yeast agglutination assay. Notably, two of these genes (lp_0373, lp_1229) encode LPXTG-containing surface proteins and were therefore considered highly likely candidates for the mannoside specific adhesin function.

Correct biogenesis of these LPXTG-containing surface proteins is strictly dependent on the sortase enzyme (encoded by the *srtA* gene; lp_0514) that catalyzes the cross-linking of these proteins to the cell-wall. In contrast to several other Gram-positive species, the *L. plantarum* genome encodes only a single sortase enzyme. Previously, *srtA* mutants of *L. plantarum* have been constructed in the inventors laboratory (unpublished). Evaluation of the *srtA* mutant derivatives of *L. plantarum* strains WCFS1 and 299v in the yeast agglutination assay revealed that these mutant had completely lost the capability to mediate yeast agglutination (Figure 1). These findings strongly supported the postulated mannose-specific adhesion properties of the two (either one of the two, or both) LPXTG-containing surface proteins identified using the biodiversity database (lp_0373, lp_1229).

### Example 3 - Validation of lp 1229 as the mannose-adhesin encoding gene,

The experimental approach to validate the potential role of lp_0373 and / or lp_1229 encompassed both the construction of knock-out mutant strains and overexpression mutants in *L. plantarum* WCFS1. Mutant derivatives of *L. plantarum* WCFS1 that lack a functional copy of either lp_0373 or lp_1229 were constructed by double-cross over gene replacement strategy. Knock-out suicide plasmid constructions were performed with *E. coli* as cloning host. Molecular biology techniques such as DNA manipulations and transformation *of E. coli* were essentially performed according to standard procedures (Sambrook, J., Fritsch, E.F., and Maniatis, T. (1989) Molecular cloning: a laboratory manual, 2nd ed. Cold Spring Harbor, NY: Cold Spring Harbor Laboratory Press.). Transformation of plasmid DNA to *L. plantarum* was performed using electroporation (Ferain, T., Garmyn, D., Bernard, N., Hols, P., and Delcour, J. (1994) Lactobacillus plantarum ldhL gene: overexpression and deletion. J Bacteriol 176: 596-601.) Plasmid DNA was isolated from *E. coli* using Jetstar columns following the manufacturer's instructions (Genomed GmbH, Bad Oeynhausen, Germany).

Primers used in this study are listed in Table 2 and were purchased from Proligo France SAS (Paris, France). Restriction endonucleases, *Taq* and Pfx polymerase and T4 DNA ligase were used as recommended by the manufacturer (Gibco BRL Life Technologies, Gaithersburg, Maryland, USA; Invitrogen, Breda, The Netherlands; New England BioLabs, Beverly, MA, USA). Plasmids used or constructed in this study are listed in Table 3.

### 3.1 Construction of lp 0373 and lp 1229 gene replacement mutants

*Lactobacillus plantarum* WCFS1 chromosomal DNA was used as a template to amplify the 5'- and 3'- flanking regions of the two candidate genes, using the primer combinations lp_0372F / lp_0372R, lp_0374F / lp_0374R, lp_1227F / lp_1227R and lp_1230F / lp_1230R, respectively (Table 2). If necessary, PCR-products were digested with *Bsa*HI (sites introduced in primers). These PCR-products (~0.8 kb) were ligated into the mutagenesis vector pNZ7101 after digestion with *Sma*I or *Pvu*II and *Bsa*HI*,* respectively. The resulting plasmids contain a chloramphenicol resistance cassette (*cat*) between the flanking regions of lp_0373 or lp_1229, respectively, as well as a erythromycin resistance gene. These vectors, designated pAL0373 and pAL1229, were used for stable double cross-over gene replacements in the chromosomal DNA of *L*. *plantarum.* Therefore, they were introduced into *L. plantarum* WCFS1 by electroporation. Integrants were selected primarily by plating on MRS agar containing 7 µg/ml chloramphenicol and double cross over candidate colonies were identified by selection of erythromycin sensitivity, as evaluated by transfer of colonies to MRS plates containing 25 µg/ml erythromycin. The anticipated double cross over gene replacement of lp_1229 and lp_0373 by the chloramphenicol resistance encoding *cat* gene was verified by PCR and Southern blot analysis.

### 3.2 Construction of lp 1229 and lp 0373 overexpression mutants

The two candidate genes lp_9373 and lp_1229 were amplified by PCR using *L. plantarum* WCFS1 genomic DNA and the primer combinations lp_0373F / lp_0373R or lp_1229F / lp_1229R. PCR-products of approximately 3.1 and 3.7 kb, respectively, were cloned into a pCR-Blunt vector (Invitrogen, Breda, The Netherlands) which subsequently was digested with *Xho*I and *Not*I (sites integrated in primers). Fragments consisting of lp_0373 and lp_1229, respectively, were subsequently ligated into expression vector pJL22. This vector contains a constitutive promoter, located within the 100bp fragment upstream of lp_1144 of *L. plantarum* WCFS1 cloned into the pNZ273 *gusA* promoter probe vector (Platteeuw C, Simons G, de Vos WM. Appl Environ Microbiol. 1994 60:587-593). The *gusA* gene of this expression plasmid was removed by digestion with *Xho*I and *Not*I digestion and replaced by lp_0373 or lp_1229, which were obtained as *Not*I*-Xho*I fragments from the corresponding PCR-BLUNT clones, yielding pAW002 and pAW005, respectively. These high-copy plasmids were transformed into *L. plantarum* WCFS1, resulting in constitutive overexpression of the genes cloned.

### 3.3 Functional analysis of mutants constructed

The constructed mutant derivatives of strain WCFS1 displayed no obvious changes in bacterial phenotype nor in apparent growth or viability differences as compared to the wildtype strain.

To investigate the importance of the two candidate genes for mannose-specific adhesion, the constructed mutant strains of *L. plantarum* WCFS1 were subsequently again tested on their agglutination capacity. Results are shown in Figure 2. Concerning lp_0373, no involvement in mannose-specific adhesion could be concluded as both mutant strains of *L. plantarum* WCFS1 either lacking or overexpressing lp_0373 showed the same behaviour in the agglutination assay as the wildtype strain. In conclusion, the protein encoded by this gene is not necessary for the capacity to agglutinate *S. cerevisiae.* In contrast, results obtained with the lp_1229 knock-out and overexpression mutants in the agglutination assay establish the involvement of lp_1229 in mannose-specific adhesion. The knock-out mutation of this gene resulted in a complete loss of the ability of *L. plantarum* WCFS1 to agglutinate *S*. *cerevisiae.* Moreover, overproduction of lp_1229 resulted in a significant increase of the yeast agglutination titre. Similar to what had been observed before, this increased agglutination efficiency displayed by the lp_1229 overproducing mutant could be completely abolished by the addition of methyl-α-D-mannopyranoside. These results clearly establish a direct role of the protein encoded by lp_1229 in mannose specific agglutination of yeast cells. These results show that lp_1229 encodes the mannoside-specific adhesin of *L. plantarum,* which is involved in the reported *L. plantarum* mediated inhibition of EPEC infection.

**Table 2: primers used for knock-out and overexpression constructs of Ip_1229, and lp_0373.**

| | |
|---|---|
| lp_0372F | 5'-AAGGAACTTCTGATTGGGCC-3' |
| lp_0372R | 5'-CCATTACGATGATGTTTCCC-3' |
| lp_0374F | |
| lp_0374R | 5'-CGTGTTTCTTGGAAGACTTC-3' |
| lp_1227F | 5'-TATGCAGAACTCCATGACTG-3' |
| lp_1227R | 5'-TAGTGTGTTTTATGCTCTCC-3' |
| lp_1230F | |
| lp_1230R | 5'-CATCACTCGACATGTCTTGC-3' |
| lp_0373F | |
| lp_0373R | |
| lp_1229F | |
| lp_1229R | 5'-ACGGGCCTCGAGGCCCTACTCTTTGTGCT-3' |
| | |

**Table 3: plasmids used in this study**

| | | |
|---|---|---|
| pNZ7101 | Cm^{R} Ery^{R}, vector for construction of L. *plantarum* gene replacement mutants, dual selection suicide vector for *L. plantarum,* | Peter Bron, unpublished |
| **pAL0373** | Cm^{R} Ery^{R}, pNZ7101 derivative containing flanking regions of *L. plantarum* WCFS 1 lp_0373 | this work |
| **pAL1229** | Cm^{R} Ery^{R}, pNZ7101 derivative containing flanking regions of *L. plantarum* WCFS1 lp_1229 | this work |
| pCR-Blunt | Kan^{R}, cloning vector | Invitrogen |
| **pCR-Blunt 0373** | Kan^{R}, pCR-Blunt derivative containing *L. plantarum* WCFS1 1p_0373 | this work |
| **pCR-Blunt 1229** | Kan^{R}, pCR-Blunt derivative containing *L. plantarum* WCFS1 lp_1229 | this work |
| **pJL22** | Cm^{R}, vector containing constitutive promoter upstream of GUS reporter gene; expression vector for *L. plantarum,* derived from pNZ123 | unpublished |
| **pAW002** | Cm^{R}, **pJL22** derivative containing *L. plantarum* WCFS1 lp_0373 | this work |
| **pAW005** | Cm^{R}, **pJL22** derivative containing *L. plantarum* WCFS1 lp_1229 | this work |

| | | |
|---|---|---|
| Cm^{R}, chloramphenicol resistant; Ery^{R}, erythromycin resistant; Kan^{R}, kanamycin resistant | | |

### Example 4 - LP 1229, protein analysis

LP_1229 is a high molecular weight surface protein (1010 amino acids) that has a multi-domain structure, which is typical for cell-surface proteins. Moreover, LP_1229 contains several repeated domains, present in both the N- and C-terminal parts of the protein. Homology searches reveals the similarity of LP_1229 to several surface proteins of different lactobacilli and staphylococci, and includes a protein of *Lactobacillus reuteri* that has been shown to be involved in mucus binding properties displayed by this species (Roos and Jonsson 2002). However, the similarities found are limited to specific domains of the LP_1229 protein and are particularly prominent in the aforementioned repeated domains.

These results show that 1p_1229 is responsible for the mannose-specific adhesion phenotype and therefore elucidates the underlying molecular mechanism of a specific health-benefit mediated by *L. plantarum* and the mutants constructed (both knock-out and overexpression) and provides the tools to modify and exploit this gene, protein and phenotype *in vivo.* The findings also allow for the first time to screen strains and strain collections for the presence and or expression of this functional property and the selection of probiotic strains.

## Claims

1. An isolated polypeptide having an amino acid sequence that has at least 60% sequence identity with the amino acid sequence of SEQ ID NO. 1 and is capable of binding mannose, or a fragment thereof capable of binding mannose, for use as a medicament.

2. A host cell comprising a nucleic acid molecule comprising a nucleotide sequence encoding a functional mannose-specific adhesin, whereby the nucleotide sequence is selected from the group consisting of:
(a) nucleotide sequences encoding a polypeptide comprising an amino acid sequence that has at least 60 % sequence identity with the amino acid sequence of SEQ ID NO. 1;
(b) nucleotide sequences comprising a nucleotide sequence that has at least 60% sequence identity with the nucleotide sequence of SEQ ID NO. 2;
(c) nucleotide sequences the complementary strand of which hybridises to a nucleic acid molecule sequence of (a) or (b) under stringent hybridisation conditions;
(d) nucleotide sequences the sequence of which differs from the sequence of a nucleic acid molecule of (c) due to the degeneracy of the genetic code,
or a nucleotide sequence as defined in a)-d) encoding a fragment of the mannose-specific adhesin capable of binding mannose, or a chimeric gene or a vector comprising such nucleic acid molecule, for use as a medicament.

3. An isolated polypeptide having an amino acid sequence that has at least 60% sequence identity with the amino acid sequence of SEQ ID NO. 1 and is capable of_binding mannose, or a fragment thereof capable of binding mannose, for use as a medicament for treating or preventing a bacterial infection of the gastrointestinal tract, the urinary tract, or the vagina.

4. A host cell comprising a nucleic acid molecule comprising a nucleotide sequence encoding a functional mannose-specific adhesin, whereby the nucleotide sequence is selected from the group consisting of:
(a) nucleotide sequences encoding a polypeptide comprising an amino acid sequence that has at least 60 % sequence identity with the amino acid sequence of SEQ ID NO. 1;
(b) nucleotide sequences comprising a nucleotide sequence that has at least 60% sequence identity with the nucleotide sequence of SEQ ID NO. 2;
(c) nucleotide sequences the complementary strand of which hybridises to a nucleic acid molecule sequence of (a) or (b) under stringent hybridisation conditions;
(d) nucleotide sequences the sequence of which differs from the sequence of a nucleic acid molecule of (c) due to the degeneracy of the genetic code,
or a nucleotide sequence as defined in a)-d) encoding a fragment of the mannose-specific adhesin capable of binding mannose, or a chimeric gene or vector comprising such nucleic acid molecule, for use as a medicament for treating or preventing a bacterial infection of the gastrointestinal tract, the urinary tract, or the vagina.

5. Use of an isolated polypeptide having an amino acid sequence that has at least 60% sequence identity with the amino acid sequence of SEQ ID NO. 1 and is capable of binding mannose, or a fragment thereof capable of binding mannose, in the preparation of a medicament for treating or preventing a bacterial infection of the gastrointestinal tract, the urinary tract, or the vagina.

6. Use of a host cell comprising a nucleic acid molecule comprising a nucleotide sequence encoding a functional mannose-specific adhesin, whereby the nucleotide sequence is selected from the group consisting of:
(a) nucleotide sequences encoding a polypeptide comprising an amino acid sequence that has at least 60 % sequence identity with the amino acid sequence of SEQ ID NO. 1;
(b) nucleotide sequences comprising a nucleotide sequence that has at least 60% sequence identity with the nucleotide sequence of SEQ ID NO. 2;
(c) nucleotide sequences the complementary strand of which hybridises to a nucleic acid molecule sequence of (a) or (b) under stringent hybridisation conditions;
(d) nucleotide sequences the sequence of which differs from the sequence of a nucleic acid molecule of (c) due to the degeneracy of the genetic code,
or a nucleotide sequence as defined in a)-d) encoding a fragment of the mannose-specific adhesin capable of binding mannose, or a chimeric gene or a vector comprising such nucleic acid molecule, in the preparation of a medicament for treating or preventing a bacterial infection of the gastrointestinal tract, the urinary tract, or the vagina.

7. Composition comprising an isolated polypeptide having an amino acid sequence that has at least 60% sequence identity with the amino acid sequence of SEQ ID NO. 1 and is capable of binding mannose, or a fragment thereof capable of binding mannose, or a host cell comprising a nucleic acid molecule comprising a nucleotide sequence encoding a mannose-specific adhesin, whereby the nucleotide sequence is selected from the group consisting of:
(a) nucleotide sequences encoding a polypeptide comprising an amino acid sequence that has at least 60 % sequence identity with the amino acid sequence of SEQ ID NO. 1;
(b) nucleotide sequences comprising a nucleotide sequence that has at least 60% sequence identity with the nucleotide sequence of SEQ ID NO. 2;
(c) nucleotide sequences the complementary strand of which hybridises to a nucleic acid molecule sequence of (a) or (b) under stringent hybridisation conditions;
(d) nucleotide sequences the sequence of which differs from the sequence of a nucleic acid molecule of (c) due to the degeneracy of the genetic code,
or a nucleotide sequence as defined in a)-d) encoding a fragment of the mannose-specific adhesin capable of binding mannose, or a chimeric gene or a vector comprising such nucleic acid molecule, and a pharmaceutically or physiologically acceptable carrier.

8. Food composition comprising an isolated polypeptide having an amino acid sequence that has at least 60% sequence identity with the amino acid sequence of SEQ ID NO. 1 and is capable of binding mannose, or a fragment thereof capable of binding mannose, or a host cell comprising a nucleic acid molecule comprising a nucleotide sequence encoding a mannose-specific adhesin, whereby the nucleotide sequence is selected from the group consisting of:
(e) nucleotide sequences encoding a polypeptide comprising an amino acid sequence that has at least 60 % sequence identity with the amino acid sequence of SEQ ID NO. 1;
(f) nucleotide sequences comprising a nucleotide sequence that has at least 60% sequence identity with the nucleotide sequence of SEQ ID NO. 2;
(g) nucleotide sequences the complementary strand of which hybridises to a nucleic acid molecule sequence of (a) or (b) under stringent hybridisation conditions;
(h) nucleotide sequences the sequence of which differs from the sequence of a nucleic acid molecule of (c) due to the degeneracy of the genetic code,
or a nucleotide sequence as defined in a)-d) encoding a fragment of the mannose-specific adhesin capable of binding mannose, or a chimeric gene or a vector comprising such nucleic acid molecule.

9. Chimeric gene comprising a nucleic acid molecule comprising a nucleotide sequence encoding a mannose-specific adhesion capable of blinding mannose, whereby the nucleotide sequence is selected from the group consisting of:
(a) nucleotide sequences encoding a polypeptide comprising an amino acid sequence that has at least 60 % sequence identity with the amino acid sequence of SEQ ID NO. 1;
(b) nucleotide sequences comprising a nucleotide sequence that has at least 60% sequence identity with the nucleotide sequence of SEQ ID NO. 2;
(c) nucleotide sequences the complementary strand of which hybridises to a nucleic acid molecule sequence of (a) or (b) under stringent hybridisation conditions;
(d) nucleotide sequences the sequence of which differs from the sequence of a nucleic acid molecule of (c) due to the degeneracy of the genetic code,
or a nucleotide sequence as defined in a)-d) encoding a fragment of the mannose-specific adhesin capable of binding mannose.

10. A vector comprising a chimeric gene according to claim 8.

11. A recombinant host cell comprising a nucleic acid molecule comprising a nucleotide sequence encoding a functional mannose-specific adhesin, whereby the nucleotide sequence is selected from the group consisting of:
(a) nucleotide sequences encoding a polypeptide comprising an amino acid sequence that has at least 60 % sequence identity with the amino acid sequence of SEQ ID NO. 1;
(b) nucleotide sequences comprising a nucleotide sequence that has at least 60% sequence identity with the nucleotide sequence of SEQ ID NO. 2;
(c) nucleotide sequences the complementary strand of which hybridises to a nucleic acid molecule sequence of (a) or (b) under stringent hybridisation conditions;
(d) nucleotide sequences the sequence of which differs from the sequence of a nucleic acid molecule of (c) due to the degeneracy of the genetic code,
or a nucleotide sequence as defined in a)-d) encoding a fragment of the mannose-specific adhesin capable of binding mannose, or a chimeric gene according to claim 9 or a vector according to claim 10.

12. A method for determining whether a bacterium has the ability to bind mannose, the method comprising determining the presence or absence of a nucleotide sequence as defined in claim 2, the presence of the nucleotide sequence being indicative of the ability to bind mannose.

## Patentansprüche

1. Isoliertes Polypeptid mit einer Aminosäuresequenz, die zumindest 60% Sequenzidentität mit der Aminosäuresequenz von SEQ ID Nr. 1 hat und zur Mannosebindung fähig ist oder ein zur Mannosebindung fähiges Fragment davon zur Verwendung als Arzneimittel.

2. Wirtszelle umfassend ein Nukleinsäuremolekül umfassend eine für ein funktionelles Mannose-spezifisches Adhäsin kodierende Nukleotidsequenz, wobei die Nukleotidsequenz aus der Gruppe bestehend aus
a) Nukleotidsequenzen, die für ein Polypeptid kodieren, das eine Aminosäuresequenz mit zumindest 60% Sequenzidentität mit der Aminosäuresequenz von SEQ ID Nr. 1 umfasst,
b) Nukleodidsequenzen umfassend eine Nukleotidsequenz mit zumindest 60% Sequenzidentität mit der Nukleotidsequenz von SEQ ID Nr. 2,
c) Nukleotidsequenzen, deren komplementärer Strang unter stringenten Hybridisierungsbedingungen an ein Nukleinsäuremolekül mit der Sequenz von a) oder b) hybridisiert,
d) Nukleotidsequenzen, deren Sequenz sich von der Sequenz eines Nukleinsäuremoleküls von c) infolge der Degeneration des genetischen Codes unterscheidet,
ausgewählt ist oder eine Nukleotidsequenz wie in a) bis d) definiert, die für ein Fragment des zur Mannosebindung fähigen Mannose-spezifischen Adhäsins kodiert oder ein chimäres Gen oder ein solche Nukleinsäuremoleküle umfassender Vektor zur Verwendung als Arzneimittel.

3. Isoliertes Polypeptid mit einer Aminosäuresequenz, die zumindest 60% Sequenzidentität mit der Aminosäuresequenz von SEQ ID Nr. 1 hat und zur Mannosebindung fähig ist oder ein zur Mannosebindung fähiges Fragment davon zur Verwendung als Arzneimittel zur Behandlung oder Verhütung einer bakteriellen Infektion des Gastrointestinaltrakts, der Harnwege oder der Vagina.

4. Wirtszelle umfassend ein Nukleinsäuremolekül umfassend eine für ein funktionelles Mannose-spezifisches Adhäsin kodierende Nukleotidsequenz, wobei die Nukleotidsequenz aus der Gruppe bestehend aus
a) Nukleotidsequenzen, die für ein Polypeptid kodieren, das eine Aminosäuresequenz mit zumindest 60% Sequenzidentität mit der Aminosäuresequenz von SEQ ID Nr. 1 umfasst,
b) Nukleodidsequenzen umfassend eine Nukleotidsequenz mit zumindest 60% Sequenzidentität mit der Nukleotidsequenz von SEQ ID Nr. 2,
c) Nukleotidsequenzen, deren komplementärer Strang unter stringenten Hybridisierungsbedingungen an die Sequenz eines Nukleinsäuremoleküls von a) oder b) hybridisiert,
d) Nukleotidsequenzen, deren Sequenz sich von der Sequenz eines Nukleinsäuremoleküls von c) infolge der Degeneration des genetischen Codes unterscheidet,
ausgewählt ist oder eine für ein wie in a) bis d) definiertes Fragment des zur Mannosebindung fähigen Mannose-spezifischen Adhäsins kodierende Nukleotidsequenz oder ein chimäres Gen oder einen Vektor, die ein solches Nukleinsäuremolekül umfassen, zur Verwendung als Arzneimittel zur Behandlung oder Verhütung einer bakteriellen Infektion des Gastrointestinaltrakts, der Harnwege oder der Vagina.

5. Verwendung eines isolierten Polypeptids mit einer Aminosäuresequenz, die zumindest 60% Sequenzidentität mit der Aminosäuresequenz von SEQ ID Nr. 1 hat und zur Mannosebindung fähig ist oder ein zur Mannosebindung fähiges Fragment davon zur Herstellung eines Arzneimittels zur Behandlung oder Verhütung einer bakteriellen Infektion des Gastrointestinaltrakts, der Harnwege oder der Vagina.

6. Verwendung einer Wirtszelle umfassend ein Nukleinsäuremolekül umfassend eine für ein funktionelles Mannose-spezifisches Adhäsin kodierende Nukleotidsequenz, wobei die Nukleotidsequenz aus der Gruppe bestehend aus
a) Nukleotidsequenzen, die für ein Polypeptid kodieren, das eine Aminosäuresequenz mit zumindest 60% Sequenzidentität mit der Aminosäuresequenz von SEQ ID Nr. 1 umfasst,
b) Nukleodidsequenzen umfassend eine Nukleotidsequenz mit zumindest 60% Sequenzidentität mit der Nukleotidsequenz von SEQ ID Nr. 2,
c) Nukleotidsequenzen, deren komplementärer Strang unter stringenten Hybridisierungsbedingungen an die Sequenz eines Nukleinsäuremoleküls von a) oder b) hybridisiert,
d) Nukleotidsequenzen, deren Sequenz sich von der Sequenz eines Nukleinsäuremoleküls von c) infolge der Degeneration des genetischen Codes unterscheidet,
ausgewählt ist oder eine für ein wie in a) bis d) definiertes Fragment des zur Mannosebindung fähigen Mannose-spezifischen Adhäsins kodierende Nukleotidsequenz oder ein chimäres Gen oder einen Vektor, die ein solches Nukleinsäuremolekül umfassen, zur Verwendung als Arzneimittel zur Behandlung oder Verhütung einer bakteriellen Infektion des Gastrointestinaltrakts, der Harnwege oder der Vagina.

7. Zusammensetzung umfassend ein isoliertes Polypeptid mit einer Aminosäuresequenz, die zumindest 60% Sequenzidentität mit der Aminosäuresequenz von SEQ ID Nr. 1 hat und zur Mannosebindung fähig ist oder ein zur Mannosebindung fähiges Fragment davon oder eine Wirtszelle umfassend ein Nukleinsäuremolekül umfassend eine für ein Mannose-spezifisches Adhäsin kodierende Nukleotidsequenz, wobei die Nukleotidsequenz aus der Gruppe bestehend aus
a) Nukleotidsequenzen, die für ein Polypeptid kodieren, das eine Aminosäuresequenz mit zumindest 60% Sequenzidentität mit der Aminosäuresequenz von SEQ ID Nr. 1 umfasst,
b) Nukleodidsequenzen umfassend eine Nukleotidsequenz mit zumindest 60% Sequenzidentität mit der Nukleotidsequenz von SEQ ID Nr. 2,
c) Nukleotidsequenzen, deren komplementärer Strang unter stringenten Hybridisierungsbedingungen an die Sequenz eines Nukleinsäuremoleküls von a) oder b) hybridisiert,
d) Nukleotidsequenzen, deren Sequenz sich von der Sequenz eines Nukleinsäuremoleküls von c) infolge der Degeneration des genetischen Codes unterscheidet,
ausgewählt ist oder eine für ein wie in a) bis d) definiertes Fragment des zur Mannosebindung fähigen Mannose-spezifischen Adhäsins kodierende Nukleotidsequenz oder ein chimäres Gen oder einen Vektor, die ein solches Nukleinsäuremolekül umfassen, und einen pharmazeutisch oder physiologisch annehmbaren Träger.

8. Nahrungszusammensetzung umfassend ein isoliertes Polypeptid mit einer Aminosäuresequenz mit zumindest 60% Sequenzidentität mit der Aminosäuresequenz von SEQ ID Nr. 1 hat und zur Mannosebindung fähig ist oder eine Wirtszelle umfassend ein Nukleinsäuremolekül, das eine für ein Mannose-spezifisches Adhäsin kodierende Nukleotidsequenz umfasst, wobei die Nukleotidsequenz aus der Gruppe bestehend aus
a) Nukleotidsequenzen, die für ein Polypeptid kodieren, das eine Aminosäuresequenz mit zumindest 60% Sequenzidentität mit der Aminosäuresequenz von SEQ ID Nr. 1 umfasst,
b) Nukleodidsequenzen umfassend eine Nukleotidsequenz mit zumindest 60% Sequenzidentität mit der Nukleotidsequenz von SEQ ID Nr. 2,
c) Nukleotidsequenzen, deren komplementärer Strang unter stringenten Hybridisierungsbedingungen an die Sequenz eines Nukleinsäuremoleküls von a) oder b) hybridisiert,
d) Nukleotidsequenzen, deren Sequenz sich von der Sequenz eines Nukleinsäuremoleküls von c) infolge der Degeneration des genetischen Codes unterscheidet,
ausgewählt ist oder eine für ein wie in a) bis d) definiertes Fragment des zur Mannosebindung fähigen Mannose-spezifischen Adhäsins kodierende Nukleotidsequenz oder ein chimäres Gen oder einen Vektor, die ein solches Nukleinsäuremolekül umfassen.

9. Chimäres Gen umfassend ein Nukleinsäuremolekül umfassend eine für ein zur Mannose-spezifischen Bindung fähiges Adhäsin kodierende Nukleotidsequenz, wobei die Nukleotidsequenz aus der Gruppe bestehend aus
a) Nukleotidsequenzen, die für ein Polypeptid kodieren, das eine Aminosäuresequenz mit zumindest 60% Sequenzidentität mit der Aminosäuresequenz von SEQ ID Nr. 1 umfasst,
b) Nukleodidsequenzen umfassend eine Nukleotidsequenz mit zumindest 60% Sequenzidentität mit der Nukleotidsequenz von SEQ ID Nr. 2,
c) Nukleotidsequenzen, deren komplementärer Strang unter stringenten Bedingungen an die Sequenz eines Nukleinsäuremoleküls von a) oder b) hybridisiert,
d) Nukleotidsequenzen, deren Sequenz sich von der Sequenz eines Nukleinsäuremoleküls von c) infolge der Degeneration des genetischen Codes unterscheidet,
ausgewählt ist oder eine für ein wie in a) bis d) definiertes Fragment des Mannose-spezifischen zur Mannosebindung fähigen Adhäsins kodierende Nukleotidsequenz.

10. Ein ein chimäres Gen nach Anspruch 8 umfassender Vektor.

11. Rekombinante Wirtszelle umfassend ein Nukleinsäuremolekül umfassend eine für ein funktionelles Mannose-spezifisches Adhäsin kodierende Nukleotidsequenz, wobei die Nukleotidsequenz aus der Gruppe bestehend aus
a) Nukleotidsequenzen, die für ein Polypeptid kodieren, das eine Aminosäuresequenz mit zumindest 60% Sequenzidentität mit der Aminosäuresequenz von SEQ ID Nr. 1 umfasst,
b) Nukleodidsequenzen umfassend eine Nukleotidsequenz mit zumindest 60% Sequenzidentität mit der Nukleotidsequenz von SEQ ID Nr. 2,
c) Nukleotidsequenzen, deren komplementärer Strang unter stringenten Hybridisierungsbedingungen an die Sequenz eines Nukleinsäuremoleküls von a) oder b) hybridisiert,
d) Nukleotidsequenzen, deren Sequenz sich von der Sequenz eines Nukleinsäuremoleküls von c) infolge der Degeneration des genetischen Codes unterscheidet,
ausgewählt ist oder eine für ein wie in a) bis d) definiertes Fragment des Mannose-spezifischen, zur Mannosebindung fähigen Adhäsins kodierende Nukleotidsequenz oder ein chimäres Gen nach Anspruch 9 oder einen Vektor nach Anspruch 10.

12. Verfahren zur Bestimmung, ob ein Bakterium die Fähigkeit zur Mannosebindung hat, wobei das Verfahren die Feststellung des Vorliegens oder des Fehlens einer in Anspruch 2 definierten Nukleotidsequenz umfasst, wobei das Vorliegen der Nukleotidsequenz auf die Fähigkeit zur Mannosebindung hinweist.

## Revendications

1. Polypeptide isolé ayant une séquence d'acides aminés qui présente une identité de séquence d'au moins 60 % avec la séquence d'acides aminés de SEQ ID NO : 1 et est capable de se lier au mannose, ou un fragment de celui-ci capable de se lier au mannose, pour utilisation comme médicament.

2. Cellule hôte comprenant une molécule d'acide nucléique qui comprend une séquence nucléotidique codant pour une adhésine fonctionnelle spécifique du mannose, la séquence nucléotidique étant choisie dans le groupe constitué par :
(a) des séquences nucléotidiques codant pour un polypeptide comprenant une séquence d'acides aminés qui présente une identité de séquence d'au moins 60 % avec la séquence d'acides aminés de SEQ ID NO : 1 ;
(b) des séquences nucléotidiques comprenant une séquence nucléotidique qui présente une identité de séquence d'au moins 60 % avec la séquence nucléotidique de SEQ ID NO : 2 ;
(c) des séquences nucléotidiques dont le brin complémentaire s'hybride à une séquence de molécule d'acide nucléique de (a) ou (b) dans des conditions d'hybridation stringentes ;
(d) des séquences nucléotidiques dont la séquence diffère de la séquence d'une molécule d'acide nucléique de (c) en raison de la dégénérescence du code génétique,
ou une séquence nucléotidique telle que définie dans les points a) à d) codant pour un fragment de l'adhésine spécifique du mannose capable de se lier au mannose, ou un gène chimère ou un vecteur qui comprend une telle molécule d'acide nucléique, pour utilisation comme médicament.

3. Polypeptide isolé ayant une séquence d'acides aminés qui présente une identité de séquence d'au moins 60 % avec la séquence d'acides aminés de SEQ ID NO : 1 et est capable de se lier au mannose, ou un fragment de celui-ci capable de se lier au mannose, pour utilisation comme médicament dans le traitement ou la prévention d'une infection bactérienne du tractus gastro-intestinal, du tractus génito-urinaire, ou du vagin.

4. Cellule hôte comprenant une molécule d'acide nucléique qui comprend une séquence nucléotidique codant pour une adhésine fonctionnelle spécifique du mannose, la séquence nucléotidique étant choisie dans le groupe constitué par :
(a) des séquences nucléotidiques codant pour un polypeptide comprenant une séquence d'acides aminés qui présente une identité de séquence d'au moins 60 % avec la séquence d'acides aminés de SEQ ID NO : 1 ;
(b) des séquences nucléotidiques comprenant une séquence nucléotidique qui présente une identité de séquence d'au moins 60 % avec la séquence nucléotidique de SEQ ID NO : 2 ;
(c) des séquences nucléotidiques dont le brin complémentaire s'hybride à une séquence de molécule d'acide nucléique de (a) ou (b) dans des conditions d'hybridation stringentes ;
(d) des séquences nucléotidiques dont la séquence diffère de la séquence d'une molécule d'acide nucléique de (c) en raison de la dégénérescence du code génétique,
ou une séquence nucléotidique telle que définie dans les points a) à d) codant pour un fragment de l'adhésine spécifique du mannose capable de se lier au mannose, ou un gène chimère ou un vecteur qui comprend une telle molécule d'acide nucléique, pour utilisation comme médicament dans le traitement ou la prévention d'une infection bactérienne du tractus gastro-intestinal, du tractus génito-urinaire, ou du vagin.

5. Utilisation d'un polypeptide isolé ayant une séquence d'acides aminés qui présente une identité de séquence d'au moins 60 % avec la séquence d'acides aminés de SEQ ID NO : 1 et est capable de se lier au mannose, ou un fragment de celui-ci capable de se lier au mannose, dans la préparation d'un médicament destiné au traitement ou à la prévention d'une infection bactérienne du tractus gastro-intestinal, du tractus génito-urinaire, ou du vagin.

6. Utilisation d'une cellule hôte comprenant une molécule d'acide nucléique qui comprend une séquence nucléotidique codant pour une adhésine fonctionnelle spécifique du mannose, la séquence nucléotidique étant choisie dans le groupe constitué par :
(a) des séquences nucléotidiques codant pour un polypeptide comprenant une séquence d'acides aminés qui présente une identité de séquence d'au moins 60 % avec la séquence d'acides aminés de SEQ ID NO : 1 ;
(b) des séquences nucléotidiques comprenant une séquence nucléotidique qui présente une identité de séquence d'au moins 60 % avec la séquence nucléotidique de SEQ ID NO : 2 ;
(c) des séquences nucléotidiques dont le brin complémentaire s'hybride à une séquence de molécule d'acide nucléique de (a) ou (b) dans des conditions d'hybridation stringentes ;
(d) des séquences nucléotidiques dont la séquence diffère de la séquence d'une molécule d'acide nucléique de (c) en raison de la dégénérescence du code génétique,
ou une séquence nucléotidique telle que définie dans les points a) à d) codant pour un fragment de l'adhésine spécifique du mannose capable de se lier au mannose, ou un gène chimère ou un vecteur qui comprend une telle molécule d'acide nucléique, dans la préparation d'un médicament destiné au traitement ou à la prévention d'une infection bactérienne du tractus gastro-intestinal, du tractus génito-urinaire, ou du vagin.

7. Composition comprenant un polypeptide isolé ayant une séquence d'acides aminés qui présente une identité de séquence d'au moins 60 % avec la séquence d'acides aminés de SEQ ID NO : 1 et est capable de se lier au mannose, ou un fragment de celui-ci capable de se lier au mannose, ou une cellule hôte comprenant une molécule d'acide nucléique qui comprend une séquence nucléotidique codant pour une adhésine spécifique du mannose, la séquence nucléotidique étant choisie dans le groupe constitué par :
(a) des séquences nucléotidiques codant pour un polypeptide comprenant une séquence d'acides aminés qui présente une identité de séquence d'au moins 60 % avec la séquence d'acides aminés de SEQ ID NO : 1 ;
(b) des séquences nucléotidiques comprenant une séquence nucléotidique qui présente une identité de séquence d'au moins 60 % avec la séquence nucléotidique de SEQ ID NO : 2 ;
(c) des séquences nucléotidiques dont le brin complémentaire s'hybride à une séquence de molécule d'acide nucléique de (a) ou (b) dans des conditions d'hybridation stringentes ;
(d) des séquences nucléotidiques dont la séquence diffère de la séquence d'une molécule d'acide nucléique de (c) en raison de la dégénérescence du code génétique,
ou une séquence nucléotidique telle que définie dans les points a) à d) codant pour un fragment de l'adhésine spécifique du mannose capable de se lier au mannose, ou un gène chimère ou un vecteur qui comprend une telle molécule d'acide nucléique, et un vecteur pharmaceutiquement ou physiologiquement acceptable.

8. Composition alimentaire comprenant un polypeptide isolé ayant une séquence d'acides aminés qui présente une identité de séquence d'au moins 60 % avec la séquence d'acides aminés de SEQ ID NO : 1 et est capable de se lier au mannose, ou un fragment de celui-ci capable de se lier au mannose, ou une cellule hôte comprenant une molécule d'acide nucléique qui comprend une séquence nucléotidique codant pour une adhésine spécifique du mannose, la séquence nucléotidique étant choisie dans le groupe constitué par :
(e) des séquences nucléotidiques codant pour un polypeptide comprenant une séquence d'acides aminés qui présente une identité de séquence d'au moins 60 % avec la séquence d'acides aminés de SEQ ID NO : 1 ;
(f) des séquences nucléotidiques comprenant une séquence nucléotidique qui présente une identité de séquence d'au moins 60 % avec la séquence nucléotidique de SEQ ID NO : 2 ;
(g) des séquences nucléotidiques dont le brin complémentaire s'hybride à une séquence de molécule d'acide nucléique de (a) ou (b) dans des conditions d'hybridation stringentes ;
(h) des séquences nucléotidiques dont la séquence diffère de la séquence d'une molécule d'acide nucléique de (c) en raison de la dégénérescence du code génétique,
ou une séquence nucléotidique telle que définie dans les points a) à d) codant pour un fragment de l'adhésine spécifique du mannose capable de se lier au mannose, ou un gène chimère ou un vecteur qui comprend une telle molécule d'acide nucléique.

9. Gène chimère comprenant une molécule d'acide nucléique comprenant une séquence nucléotidique codant pour une adhésine spécifique du mannose capable de se lier au mannose, la séquence nucléotidique étant choisie dans le groupe constitué par :
(a) des séquences nucléotidiques codant pour un polypeptide comprenant une séquence d'acides aminés qui présente une identité de séquence d'au moins 60 % avec la séquence d'acides aminés de SEQ ID NO : 1 ;
(b) des séquences nucléotidiques comprenant une séquence nucléotidique qui présente une identité de séquence d'au moins 60 % avec la séquence nucléotidique de SEQ ID NO : 2 ;
(c) des séquences nucléotidiques dont le brin complémentaire s'hybride à une séquence de molécule d'acide nucléique de (a) ou (b) dans des conditions d'hybridation stringentes ;
(d) des séquences nucléotidiques dont la séquence diffère de la séquence d'une molécule d'acide nucléique de (c) en raison de la dégénérescence du code génétique,
ou une séquence nucléotidique telle que définie dans les points a) à d) codant pour un fragment de l'adhésine spécifique du mannose capable de se lier au mannose.

10. Vecteur comprenant un gène chimère selon la revendication 8.

11. Cellule hôte recombinante comprenant une molécule d'acide nucléique qui comprend une séquence nucléotidique codant pour une adhésine fonctionnelle spécifique du mannose, la séquence nucléotidique étant choisie dans le groupe constitué par :
(a) des séquences nucléotidiques codant pour un polypeptide comprenant une séquence d'acides aminés qui présente une identité de séquence d'au moins 60 % avec la séquence d'acides aminés de SEQ ID NO : 1 ;
(b) des séquences nucléotidiques comprenant une séquence nucléotidique qui présente une identité de séquence d'au moins 60 % avec la séquence nucléotidique de SEQ ID NO : 2 ;
(c) des séquences nucléotidiques dont le brin complémentaire s'hybride à une séquence de molécule d'acide nucléique de (a) ou (b) dans des conditions d'hybridation stringentes ;
(d) des séquences nucléotidiques dont la séquence diffère de la séquence d'une molécule d'acide nucléique de (c) en raison de la dégénérescence du code génétique,
ou une séquence nucléotidique telle que définie dans les points a) à d) codant pour un fragment de l'adhésine spécifique du mannose capable de se lier au mannose, ou un gène chimère selon la revendication 9 ou un vecteur selon la revendication 10.

12. Procédé de détermination de la capacité d'une bactérie de se lier au mannose, le procédé comprenant la détermination de la présence ou de l'absence d'une séquence nucléotidique telle que définie dans la revendication 2, la présence de la séquence nucléotidique indiquant la capacité de liaison au mannose.


### SEQUENCE LISTING

<110> Wageningen Centre for Food Sciences
<120> Novel mannose-specific adhesins and their use
<130> P212925EP
<140> P212925EP
<141> 2003-03-23
<160> 16
<170> PatentIn version 3.1
<210> 1
   <211> 1010
   <212> PRT
   <213> Lactobacillus plantarum
<400> 1
   Met Leu Lys Lys Asp Asn Phe Gly Glu His Lys Thr His Tyr Lys Leu
   1               5                   10                  15
   Tyr Lys Cys Gly Lys Asn Trp Ala Ile Met Gly Ile Thr Leu Val Ser
               20                  25                  30
   Leu Gly Val Gly Thr Val Thr Met Thr Arg Ala Ala Ala Ala Asp Ser
           35                  40                  45
   Glu Val Thr Asn Asp Ser Ala Ser Gln His Val Thr Ser Ile Ser Thr
       50                  55                  60
   Asp Ala Ser Lys Asn Gln His Thr Ser Ser Asn Val Ile Leu Thr Asn
   65                  70                  75                  80
   Asp Asp Lys Ser Val Ser Ala Ser Ile Asn Gln Asp Ala Ser Ala Ser
                   85                  90                  95
   Val Val Asn Lys Ala Val Ser Ala Thr Ser Gln Glu Asn Ser Ser Val
               100                 105                 110
   Gln Asn Thr Ser Gln Ala Thr Ser Thr Ser Lys Gln Glu Ser Ser Ser
           115                 120                 125
   Thr Lys Asn Thr Ser Gln Thr Thr Ser Thr Ser Asn Gln Glu Ala Asn
       130                 135                 140
   Ser Ala Lys Ser Ile Asn Gln Thr Thr Arg Thr Ser Lys Gln Glu Ser
   145                 150                 155                 160
   Ser Ser Thr Lys Asn Thr Ser Gln Thr Thr Ser Thr Ser Asn Gln Glu
                   165                 170                 175
   Ala Asn Ser Ala Lys Ser Ile Asn Gln Thr Thr Arg Thr Ser Asn Gln
               180                 185                 190
   Glu Ser Ser Ser Ala Lys Asn Thr Ser Gln Thr Thr Ser Thr Ser Ser
           195                 200                 205
   Arg Lys Ile Asn Ser Thr Lys Ser Gln Ala Gln Ser Leu Thr Ile Thr
       210                 215                 220
   Thr Thr Gly Lys Ala Val Arg Ala Thr Ser Thr Ser Val Lys Lys Tyr
   225                 230                 235                 240
   Ser Thr Lys Thr Lys Val Ser Tyr Ser Thr Leu Leu Gln Gln Leu Arg
                   245                 250                 255
   Thr Ser Lys Ala Leu Ile Ser Asp Glu Ala Ala Leu Thr His Val Asp
               260                 265                 270
   Lys Asp Asn Phe Leu Lys Tyr Phe Ser Leu Asn Gly Ser Ala Thr Tyr
           275                 280                 285
   Asp Ala Lys Thr Gly Ile Val Thr Ile Thr Pro Asn Gln Asn Asn Gln
       290                 295                 300
   Val Gly Asn Phe Ser Leu Thr Ser Lys Ile Asp Met Asn Lys Ser Phe
   305                 310                 315                 320
   Thr Leu Thr Gly Gln Val Asn Leu Gly Ser Asn Pro Asn Gly Ala Asp
                   325                 330                 335
   Gly Ile Gly Phe Ala Phe His Ser Gly Asn Thr Thr Asp Val Gly Asn
               340                 345                 350
   Ala Gly Gly Asn Leu Gly Ile Gly Gly Leu Gln Asp Ala Ile Gly Phe
           355                 360                 365
   Lys Leu Asp Thr Trp Phe Asn Ser Tyr Gln Ala Pro Ser Ser Asp Lys
       370                 375                 380
   Asn Gly Ser Glu Ile Ser Ser Thr Asn Ser Asn Gly Phe Gly Trp Asn
   385                 390                 395                 400
   Gly Asp Ser Ala Asn Ala Pro Tyr Gly Thr Phe Val Lys Thr Ser Asn
                   405                 410                 415
   Gln Glu Ile Ser Thr Ala Asn Gly Ser Lys Val Gln Arg Trp Trp Ala
               420                 425                 430
   Gln Asp Thr Gly Glu Ser Gln Ala Leu Ser Lys Ala Asp Ile Asp Gly
           435                 440                 445
   Asn Phe His Asp Phe Val Val Asn Tyr Asp Gly Ala Thr Arg Thr Leu
       450                 455                 460
   Thr Val Ser Tyr Thr Gln Ala Ser Gly Lys Val Leu Thr Trp Lys Thr
   465                 470                 475                 480
   Thr Val Asp Ser Ser Tyr Gln Ala Met Ala Met Val Val Ser Ala Ser
                   485                 490                 495
   Thr Gly Ala Ala Lys Asn Leu Gln Gln Phe Lys Leu Thr Ser Phe Asp
               500                 505                 510
   Phe Gln Glu Ala Ala Thr Val Asn Val Lys Tyr Val Asp Thr Thr Gly
           515                 520                 525
   His Gln Leu Ala Gln Gly Thr Ala Asn Tyr Pro Asp Gly Ala Tyr Val
       530                 535                 540
   Asn Gly Arg Tyr Thr Thr Lys Gln Leu Ile Ile Pro Asn Tyr Arg Phe
   545                 550                 555                 560
   Ile Lys Met Asp Asp Gly Ser Val Thr Gly Thr Lys Ser Leu Asp Ala
   565 570 575
   Asn Gly Thr Leu Ile Gln Ser Gly Asp Asn Gly Thr Val Ile Tyr Val
   580 585 590
   Tyr Val Pro Glu Tyr Met Ala Ile Val Lys Thr Val Asn Glu Thr Ile
   595 600 605
   Asn Tyr Val Asp Glu Asn Gly His Ala Leu Thr Thr Ser Tyr Thr Ala
   610 615 620
   Asn Pro Ile His Ile Leu Thr Val Thr Asn Pro Val Asp Gly Thr Thr
   625 630 635 640
   Thr Thr Tyr Tyr Ser Thr Ile Thr Thr Ser Ile Glu Leu Asp Ala Thr
   645 650 655
   Thr Gly Arg Pro Val Asp Ser Gly Trp Val Leu Gly Asn Ser Gln Asp
   660 665 670
   Phe Asp Ala Val Thr Asn Pro Gln Ile Lys Gly Tyr Thr Val Thr Ser
   675 680 685
   Thr Asp Ala Pro Asn Ser Asp Leu Gln His Val Ser Ala Gln Thr Val
   690 695 700
   Thr Gly Asp Ser Gly Asp Leu Glu Phe Thr Val Val Tyr Thr Lys Asn
   705 710 715 720
   Ala Pro Ile Val Thr Thr Glu Ser Lys Thr Val Asn Glu Thr Ile His
   725 730 735
   Tyr Val Tyr Thr Asp Gly Thr Thr Ala His Asp Asp Tyr Val Ala Gln
   740 745 750
   Pro Ile Thr Phe Thr Arg Thr Val Phe Thr Asp Ala Val Thr Gly Glu
   755 760 765
   Lys Thr Tyr Gly Gly Trp Ser Ala Ala Gln Gln Phe Ala Ala Val Asp
   770 775 780
   Ser Pro Ala Ile Lys Gly Tyr Thr Pro Asp Gln Ser Lys Ile Ser Thr
   785 790 795 800
   Gln Thr Val Thr Gly Asp Ser Ser Asp Leu Glu Phe Thr Ile Val Tyr
   805 810 815
   Thr Lys Asn Ala Pro Thr Val Thr Thr Glu Ser Lys Thr Val Asn Glu
   820 825 830
   Thr Ile His Tyr Val Tyr Thr Asp Gly Thr Ile Ala His Asp Asp Tyr
   835 840 845
   Val Ala Gln Pro Ile Thr Phe Thr Arg Thr Val Ser Thr Asp Ala Val
   850 855 860
   Thr Gly Glu Lys Thr Tyr Gly Gly Trp Ser Ala Ala Gln Gln Phe Ala
   865 870 875 880
   Ala Val Asp Ser Pro Ala Ile Lys Gly Tyr Thr Pro Asp Gln Ser Lys
   885 890 895
   Ile Ser Thr Gln Thr Val Thr Gly Asp Ser Ser Asp Leu Glu Phe Thr
   900 905 910
   Val Val Tyr Lys Ala Asp Ser Thr Ser Thr Lys Pro Val Lys Pro Glu
   915 920 925
   Gln Pro Thr Ile Pro Thr Thr Pro Thr Glu Pro Val Lys Pro Gly Gln
   930 935 940
   Leu Thr Thr Pro Ala Lys Pro Asp Gln Pro Met Thr Ser Asp Lys Ser
   945 950 955 960
   Val Gln Thr Ile Thr Ile Lys Phe Val Gly Gln Arg Leu Pro Gln Thr
   965 970 975
   Asn Glu Thr Asp Gln Gln His Met Thr Leu Ser Gly Leu Leu Leu Leu
   980 985 990
   Ala Met Ser Gly Leu Leu Gly Leu Leu Gly Met Ala Lys Arg Gln His
   995 1000 1005
   Lys Glu
   1010
<210> 2
   <211> 3030
   <212> DNA
   <213> Lactobacillus plantarum
<400> 2
   atgttaaaaa aggataattt tggagagcat aaaacacact ataagcttta taaatgtgga 60
   aaaaactggg ctattatggg gattacgttg gtttcattgg gggtaggtac tgttactatg 120
   acacgggccg ccgcagcaga ttccgaagtg acgaacgatt cagcatctca acatgtgacg 180
   agcatctcta ctgatgcgtc taaaaatcag catacgtcta gtaatgtgat tcttacaaat 240
   gatgataaaa gtgttagtgc atcaattaat caagacgcta gcgcgagtgt tgtcaataag 300
   gcggtcagcg caactagcca agaaaatagt agcgttcaaa atactagtca ggcaaccagt 360
   acaagcaaac aagaatccag tagtacgaaa aatactagtc agacaaccag tacaagcaat 420
   caagaagcta acagtgccaa gagtattaat caaacgaccc gtacaagcaa acaagaatcc 480
   agtagtacga aaaatactag tcagacaacc agtacaagca atcaagaagc taacagtgcc 540
   aagagtatta atcaaacgac ccgtacaagc aatcaagaat ccagtagtgc gaaaaatact 600
   agtcagacaa ccagtacaag tagtcggaag attaatagca ctaaaagcca agcccaatca 660
   ttaacaatta cgactacggg caaagcggtt cgtgctacct cgactagtgt taaaaagtac 720
   tccaccaaga caaaagtgtc atatagcaca ttgttacagc agcttcgtac aagtaaagcg 780
   ttgatctctg atgaagcggc cttgactcat gtagacaagg acaatttcct aaagtatttt 840
   agtttgaacg gatctgcaac atatgatgcc aagacgggaa ttgtaactat tacgcccaat 900
   caaaataatc aagttggtaa tttttcatta accagtaaga ttgatatgaa taaaagcttt 960
   acattaactg gtcaggtaaa tctggggtct aacccgaatg gtgcggatgg aattgggttt 1020
   gcttttcaca gtggcaatac aactgacgtg ggaaatgctg gtggtaattt aggtattggt 1080
   ggattgcaag acgctatcgg gttcaagcta gacacatggt ttaatagcta ccaagcacca
   1140
   tcatcagata aaaatgggag tgaaatctca tcaacaaatt ctaatggctt tggttggaat
   1200
   ggtgactcag ccaacgcacc atatggcacc tttgtcaaga cgagtaacca agaaatttcg
   1260
   actgcgaatg gttctaaggt acagcgatgg tgggctcaag atacaggaga gtcgcaggcg
   1320
   ttaagtaaag cggatattga tggtaacttt catgattttg tagttaacta tgatggtgct
   1380
   acaagaacgt taaccgttag ttatacgcaa gctagtggta aagtattaac ttggaagacg
   1440
   actgttgaca gttcttatca agcaatggcc atggttgtca gtgcatcaac tggtgcagct
   1500
   aaaaatttac aacaatttaa gttgactagc ttcgattttc aagaagcagc gactgtgaat
   1560
   gtaaaatatg ttgatactac tggacatcag ctagctcaag gaacagctaa ttatccagat
   1620
   ggagcgtatg tcaatggtcg gtacaccact aaacaattaa taattcccaa ttatagattt
   1680
   attaagatgg atgatggctc agttaccggc acaaaaagtt tagatgccaa tggaacctta
   1740
   atccaatcgg gtgacaatgg tactgttatc tatgtatatg tgcctgaata tatggctatt
   1800
   gtcaagacag ttaacgaaac tattaattat gttgatgaaa atggtcatgc gttgaccact
   1860
   agctacaccg ccaatcccat ccatatattg accgtgacga atccggtgga tggaacgacg
   1920
   acaacctact attcaacaat tacaacatcg atagaactag atgcgacaac tggtagacca
   1980
   gtcgattctg gatgggtatt gggtaatagt caagatttcg atgcagttac aaatccacaa
   2040
   atcaagggct atacagtaac aagtactgat gcgcctaatt cagatttgca gcatgtgtca
   2100
   gcgcagacgg ttactggtga ttctggtgac cttgaattta ccgtcgtgta caccaagaat
   2160
   gccccaatcg tgacaacgga aagtaaaacg gtcaatgaga ctattcatta tgtttatacg
   2220
   gatggaacta cagcccacga tgactatgtg gcccagccca tcacgtttac acggactgta
   2280
   tttacagatg ctgtcacggg tgaaaagaca tatgggggtt ggtcggcagc gcaacaattt
   2340
   gcggccgtgg atagtccagc aattaagggc tacacaccag atcagagcaa aattagtacg
   2400
   cagaccgtta ctggcgattc cagtgacctt gaatttacca tcgtgtacac caagaatgct
   2460
   ccaaccgtga caacggaaag taaaacggtc aatgagacta ttcattatgt ttatacggat
   2520
   ggaactatag cccatgatga ttatgtggcc cagcccatca cgtttacacg gactgtatct
   2580
   acagatgctg tcacgggtga aaagacatat gggggttggt cggcagcgca acaatttgcg
   2640
   gccgtggata gtccagcaat taagggctac acaccagatc agagcaaaat tagtacgcag
   2700
   accgttactg gtgattccag tgaccttgaa tttacggtag tttataaggc tgatagcaca
   2760
   tcaacaaaac cggttaaacc agaacaacca acgataccaa caacaccgac agaaccggtt
   2820
   aaaccaggac agctaacgac accagcaaaa ccagatcaac cgatgacatc tgataagtcg
   2880
   gttcaaacaa taaccattaa gtttgtgggt caacgattac cacaaactaa tgaaacggat
   2940
   caacaacata tgacgcttag tggtttattg ttattagcca tgagtggcct attaggtctc
   3000
   cttggtatgg ctaagcgaca gcacaaagag
   3030
<210> 3
   <211> 234
   <212> PRT
   <213> Lactobacillus plantarum
<400> 3
   Met Lys Ser Lys Gln His Pro Gly Leu Lys Trp Leu Gly Arg Gly Val
   1 5 10 15
   Phe Val Leu Leu Val Leu Val Ser Leu Ala Leu Ile Phe Asn Glu Gln
   20 25 30
   Ile Lys Ser Trp Leu Val Ser Ser Tyr Ser Pro Thr Val Thr Ala Lys
   35 40 45
   Thr Val Lys Gln Asn Thr Lys Lys Lys Ser Asp Phe Asn Phe Ser Lys
   50 55 60
   Val Lys Ser Leu Asp Phe Gln Thr Val Ala Lys Ala Arg Met Asn Lys
   65 70 75 80
   Asn Ala Ile Asn Val Ile Gly Ser Ile Ala Ile Pro Ser Val Asp Leu
   85 90 95
   Tyr Leu Pro Ile Gly Asn Gly Val Ser Asn Glu Thr Leu Ala Leu Ala
   100 105 110
   Ala Gly Thr Met Lys Ala Asn Gln Lys Met Gly Gln Gly Asn Tyr Ala
   115 120 125
   Leu Ala Gly His His Met Ile Lys His Gly Ala Leu Phe Ser Pro Leu
   130 135 140
   Tyr Tyr Lys Ser Lys Val Gly Gln Met Ile Tyr Val Ser Asp Ala Lys
   145 150 155 160
   Lys Ile Tyr Ala Tyr Lys Thr Ser Gln Arg Thr Phe Ile Lys Ala Thr
   165 170 175
   Asp Val Gln Val Ile Asp Asp Val Pro Gly Gln Lys Leu Ile Thr Leu
   180 185 190
   Ile Thr Cys Asp Lys Thr Gly Ala Gly Arg Leu Met Ile Arg Gly Lys
   195 200 205
   Tyr Glu Gln Gln Trp Ser Phe Lys Ser Ala Pro Thr Gln Val Gln Lys
   210 215 220
   Ala Phe Thr Ser His Phe Asn Asn Lys Tyr
   225 230
<210> 4
   <211> 702
   <212> DNA
   <213> Lactobacillus plantarum
<400> 4
   atgaagtcca agcaacatcc cgggttaaag tggttaggtc ggggcgtttt tgtcctgctg 60
   gtgctagtat cattggcatt aattttcaat gaacagatca agagctggtt agtgagttca 120
   tactcgccga ccgttacggc taagacggtt aagcagaata cgaagaagaa gagcgatttt 180
   aacttctcaa aggtcaagtc acttgacttt cagaccgttg ccaaggcccg catgaataag 240
   aatgcaatca acgtgattgg ctcgattgcg attccgtccg ttgatttgta cctgccaatc 300
   ggtaatggtg tcagcaatga aacgttagca ttggcggctg gcacgatgaa agctaatcag 360
   aagatggggc agggtaacta cgcgttggct ggtcaccata tgatcaagca tggtgcacta 420
   ttcagtccgt tgtactataa gagcaaggtt ggccaaatga tttatgtgag tgatgccaaa 480
   aagatttatg cgtataagac gagtcagcga acattcatta aagcaactga tgtccaagta 540
   attgatgatg ttcctggtca gaagctgatt accctaatta cctgtgataa gaccggggcg 600
   ggtcgcttaa tgattcgcgg taagtacgaa cagcaatggt cgtttaagtc ggcgccaact 660
   caggttcaga aggcctttac aagtcatttt aataacaaat at 702
<210> 5
   <211> 20
   <212> DNA
   <213> Artificial
<400> 5
   aaggaacttc tgattgggcc 20
<210> 6
   <211> 20
   <212> DNA
   <213> Artificial
<400> 6
   ccattacgat gatgtttccc 20
<210> 7
   <211> 32
   <212> DNA
   <213> Artificial
<400> 7
   aattacggcg tctcactgta gcgattacga tg
   32
<210> 8
   <211> 20
   <212> DNA
   <213> Artificial
<400> 8
   cgtgtttctt ggaagacttc
   20
<210> 9
   <211> 20
   <212> DNA
   <213> Artificial
<400> 9
   tatgcagaac tccatgactg
   20
<210> 10
   <211> 20
   <212> DNA
   <213> Artificial
<400> 10
   tagtgtgttt tatgctctcc
   20
<210> 11
   <211> 32
   <212> DNA
   <213> Artificial
<400> 11
   atttatggcg tcgtctcctt ggtatggcta ag
   32
<210> 12
   <211> 20
   <212> DNA
   <213> Artificial
<400> 12
   catcactcga catgtcttgc
   20
<210> 13
   <211> 39
   <212> DNA
   <213> Artificial
<400> 13
   aaggacagaa gcggccgcgt tagataaaag gggttaaac
   39
<210> 14
   <211> 31
   <212> DNA
   <213> Artificial
<400> 14
   acgggcctcg agatattagt cacgccctcg t
   31
<210> 15
   <211> 36
   <212> DNA
   <213> Artificial
<400> 15
   aaggacagaa gcggccgccg tgcgaaagga tagatt
   36
<210> 16
   <211> 29
   <212> DNA
   <213> Artificial
<400> 16
   acgggcctcg aggccctact ctttgtgct
   29